# FASCICULE DE BREVET EUROPEEN

(11) **EP 2 943 478 B1**
(45) Date de publication et mention de la délivrance du brevet: **02.08.2017**
(21) Numéro de dépôt: 14703109.0
(22) Date de dépôt: 07.01.2014
(51) Int. Cl.: C07D 309/40, C07C 69/618, C07C 323/20, C07C 235/34, C07C 235/38, C07D 209/16, C07D 211/06, C07D 295/192, A61Q 19/08, A61K 31/216, A61P 17/00

(54) **NOUVEAUX DÉRIVÉS DE L'ACIDE SINAPINIQUE**
NEUARTIGE DERIVATE VON SINAPINSÄURE
NOVEL DERIVATIVES OF SINAPINIC ACID

(30) Priorité: 08.01.2013 FR 1350125
(43) Date de publication de la demande: 18.11.2015
(73) Titulaire: Pharmasynthese, 76320 Saint-Pierre-Les-Elbeuf (FR)
(72) Inventeur: LE ROY, Pierre-Yves, F-91710 Vert Le Petit (FR); LE GUEN, Yves, F-75007 Paris (FR)
(74) Mandataire: Agasse, Stéphane
(86) Numéro de dépôt international: PCT/FR2014/050017
(87) Numéro de publication internationale: WO 2014/108629

(56) Documents cités:
- EP-A1- 1 437 117

## Description

La présente invention a pour objet de nouveaux dérivés de l'acide sinapinique et l'utilisation de ces dérivés pour des applications cosmétiques ou pharmaceutiques.

### Antioxydants

L'oxydation fait partie d'une réaction d'oxydoréduction qui transfère des électrons d'une substance vers un agent oxydant. Cette réaction peut produire des radicaux libres qui entraînent des réactions en chaîne. Bien que les réactions d'oxydation soient nécessaires à la vie, elles peuvent aussi être destructrices.

Le stress oxydatif a été mis en cause dans la pathogénèse de nombreuses maladies humaines. Ainsi, le stress oxydatif peut endommager, voire tuer les cellules et peut être une cause partielle du développement de plusieurs maladies dégénératives chroniques incluant cancer, dysfonctionnement cardiaque et dégénération neuronale. Il est également connu que des molécules oxydantes peuvent endommager des molécules biologiques telles que les protéines, les lipides ou l'ADN. Bien que le corps humain ait développé des outils de lutte contre les radicaux libres, le processus d'élimination n'est pas efficace à 100%.

Il est aujourd'hui largement accepté que le stress oxydatif est impliqué dans le processus de vieillissement de cellule la peau (Ames, B. N., Shigenaga, M. K. and Hagen, T. M. (1993), « Oxidants, Antioxidants, and the Degenerative Diseases of Aging », Proc. Natl. Acad. Sci. USA 90, 7915-7922). Les antioxydants sont donc susceptibles de retarder le vieillissement cutané.

Les antioxydants sont capables de stopper ces réactions en chaîne en se réduisant avec les radicaux libres, annihilant ainsi leur action. Ainsi, les plantes et les animaux utilisent et produisent de nombreux antioxydants, c'est-à-dire des molécules susceptibles de diminuer ou empêcher l'oxydation d'autres substances chimiques.

Dans le cadre du traitement cosmétique du vieillissement cutané, il est donc utile d'identifier des composés ayant une activité antioxydante.

### Elastine

L'élastine est une protéine de la famille des protéines fibreuses de type structural. L'élastine est synthétisée et sécrétée dans l'espace extracellulaire par les fibroblastes d'abord en proélastine, puis en tropoélastine.

L'élastine est la composante majeure des fibres élastiques.

L'élastine se retrouve dans le derme de la peau, celui-ci agissant en tant que soutien. Le bon fonctionnement de la peau en particulier est étroitement lié aux caractéristiques de l'élastine qui peut s'étirer jusqu'à 150% de sa longueur au repos avant de se briser.

Ainsi, elle permet aux tissus de s'étirer et de retrouver leur état initial après l'étirement, ce qui leur donne de la souplesse.

Par ailleurs, la production totale d'élastine s'arrête autour de la puberté, après quoi, la quantité d'élastine disponible diminuera avec le temps ce qui entrainera, par exemple, au cours du vieillissement, une perte d'élasticité et de tonicité du derme qui ne peut plus s'opposer aux effets de contraction des muscles sous-jacents donne lieu à l'apparition des rides.

Dans le cadre du traitement cosmétique du vieillissement cutané, il est donc utile d'identifier des composés capables d'induire une augmentation significative de la néosynthèse d'élastine.

### Collagène

Le collagène est une famille de protéines, le plus souvent présente sous forme fibrillaire. Elle est présente dans la matrice extra-cellulaire des organismes. Ces protéines ont pour fonction de conférer aux tissus une résistance mécanique à l'étirement.

Contrairement à l'élastine présente aussi dans les tissus conjonctifs, le collagène est inextensible et résiste bien à la traction. Il existe différents types de collagène selon l'organe considéré. Par exemple, le collagène de type I (représentant 90 % du collagène d'un vertébré) constitue la trame de l'os (à comparer aux armatures du béton armé), et plus généralement des tissus conjonctifs banals. Il se trouve dans les os, la peau, les tendons, la cornée et les organes internes.

La production de collagène dans la peau commence à diminuer dès l'âge de 25 ans, mais ce ralentissement s'accélère à la quarantaine, avec une perte collagénique qui pourrait tourner autour de 1 % par an.

Conséquences du ralentissement de la production de collagène et de ses altérations, la peau retient moins d'eau, devient moins souple, s'amincit et se ride.

Dans le cadre du traitement cosmétique du vieillissement cutané, il est donc utile d'identifier des composés favorisants la néosynthèse de collagène de type I (pro-collagène I).

### Progérine

Le vieillissement cutané peut également être attribué à certaines maladies bien connues, parmi lesquelles la progéria ou syndrome de Hutchinson-Gilford. Les symptômes de cette maladie se caractérisent par un vieillissement accéléré conduisant à la mort du patient.

Dans cette pathologie, la lamine-A, une protéine participant à la formation de la lamina nucléaire et impliquée dans la stabilité du noyau de la structure de la chromatine et de l'expression des gênes, est présente sous une forme tronquée appelée progérine.

D'autre part, il a été récemment démontré (C. Verdy, J.-E. Branka et N. Mekideche, « Quantitative assessment of lactate and progerine production in normal human cutaneous cells during normal ageing : effect of an alaria esculenta extract », Int. J. Cosmetic Science, 2011, 1-5) que des extraits de l'algue *Aleria esculenta,* induisant une diminution significative de la néo synthèse de progérine, montraient également un effet notable sur une activité anti-âge de la peau.

Dans le cadre du traitement cosmétique du vieillissement cutané ou du traitement thérapeutique du vieillissement cutané, notamment de la maladie de Hutchinson-Gilford, il est donc utile d'identifier des composés permettant de diminuer la néosynthèse de progérine.

### Glycerol

Le tissu adipeux (masse grasse) est un tissu conjonctif spécial dont la constitution ressemble à celle d'un tissu conjonctif, avec une substance fondamentale, des fibres et ses cellules. C'est en fait un tissu conjonctif contenant des cellules graisseuses stockant les lipides, appelées « adipocytes ».

Ces réserves de lipides sont constituées de triglycérides. Ces triglycérides sont synthétisés à l'intérieur de l'adipocyte mais l'endocytose, c'est-à-dire la diffusion de ces triglycérides à travers la membrane cytoplasmique, n'est pas facilitée. L'adipocyte va donc excréter dans le sang une lipase pour cliver les triglycérides en acide gras et glycérol qui sont facilement assimilables par l'adipocyte. Le mécanisme inverse est effectué lors de l'excrétion des réserves lipiques par l'intermédiaire d'une enzyme, la lécithine.

Dans le cadre d'un traitement cosmétique amincissant, il est donc utile d'identifier des composés permettant de diminuer le taux de glycérol dans les tissus adipeux.

### Mélano-modulateurs

L'épiderme, les cheveux et les poils sont colorés par des pigments, les mélanines, produites par des cellules spécialisées de grande taille : les mélanocytes. Ils sont situés dans la couche la plus profonde de l'épiderme. Ces pigments mélaniques servent à protéger l'épiderme et les couches profondes de la peau des agressions externes, en particulier des rayons ultraviolets. Les mélanines jouent ainsi un rôle photoprotecteur important. La biosynthèse de mélanine s'effectue selon une série complexe de réactions enzymatiques nommée mélanogénèse.

Dans le cadre d'un traitement cosmétique de blanchiment de la peau ou d'éclaircissement de la peau, il est donc utile d'identifier des composés permettant de réguler (diminuer) la synthèse de mélanine.

L'acide sinapinique, également appelé acide sinapique ou acide 3,5-diméthoxy-4-hydroxycinnamique, est un acide phénolique de formule chimique :

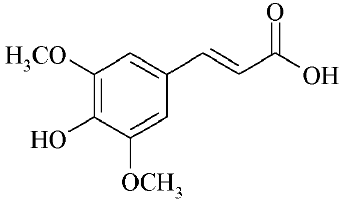

L'acide sinapinique se trouve dans une large variété de plantes, notamment dans les plantes oléagineuses, en particulier dans les graines de colza.

L'utilisation de l'acide sinapinique et de ses proches dérivés dans le domaine cosmétique est connue de l'art antérieur. Ainsi, la demande de brevet EP-A-1437117 décrit l'utilisation de l'acide sinapinique et de ses proches dérivés pour la préparation de compositions cosmétiques susceptibles d'être utilisées dans le cadre de traitement anti-âge ou anti-rides.

La demande de brevet EP-A-1967175 décrit quant à elle l'utilisation de dérivés de l'acide sinapinique comme agent blanchissant de la peau. Une activité anti-âge est également suggérée. Néanmoins, aucun résultat expérimental permettant de confirmer une telle activité n'est fourni.

Néanmoins, aucune de ces demandes de brevet ne décrit les composés selon la présente invention.

Or, il a maintenant été trouvé de nouveaux dérivés de l'acide sinapinique qui, de façon tout à fait surprenante, sont efficaces dans le cadre du traitement cosmétique du vieillissement cutané ; dans la cadre du traitement thérapeutique du vieillissement cutané, notamment dans le cadre du traitement de la maladie de Hutchinson-Gilford ; et/ou dans le cadre d'un traitement cosmétique amincissant.

La présente invention a donc pour objet un composé de formule générale (I) 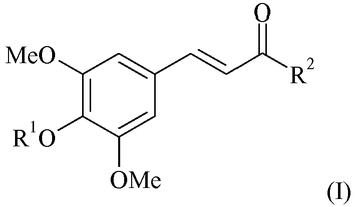 dans laquelle :
- R¹ est choisi comme étant un groupe C₂-C₆-alkyle ou un groupe -(C=O)-R³;
- R² est choisi comme étant un groupe -O-R⁴ ou -(N)R⁵R⁶ ;
- R³ est choisi comme étant un groupe C₁-C₆-alkyle ;
- R⁴ est choisi comme étant un groupe C₁₂-C₁₆-alkyle, C₁₂-C₁₆-alkényle, C₁₂-C₁₆-alkynyle, phényle, 4-pyranone, C₁-C₁₆-alkylphényle, C₂-C₁₆-alkénylphényle, C₂-C₁₆-alkynylphényle, C₃-C₆-cycloalkyle, C₁-C₁₆-alkyl-C₃-C₆-cycloalkyle, C₂-C₁₆-alkényle-C₃-C₆-cycloalkyle et C₂-C₁₆-alkynyle-C₃-C₆-cycloalkyle ; chacun de ces groupes étant éventuellement substitué par un ou plusieurs substituants choisis indépendamment les uns des autres comme étant un groupe hydroxy, amine, C₁-C₆-alkyle, C₁-C₆-hydroxyalkyle, C₁-C₆-alkoxy, C₁-C₆-alkylthio, C₁-C₆-alkylcarbonyloxy, phényle, C₁-C₆-alkoxyphényle, ou C₂-C₆-alkénylphényle éventuellement substitué par un ou plusieurs substituants choisis indépendamment les uns des autres comme étant un groupe C₁-C₆-alkyle, C₁-C₆-hydroxyalkyle, C₁-C₆-alkoxy, C₁-C₆-thioalkyle ou C₁-C₆-alkylcarbonyloxy ;
- R⁵ et R⁶ sont choisis indépendamment l'un de l'autre comme étant un atome d'hydrogène ou un groupe choisi parmi C₁-C₁₆-alkyle, C₂-C₁₆-alkényle, C₂-C₁₆-alkynyle, phényle, C₁-C₁₆-alkylphényle, C₂-C₁₆-alkénylphényle, C₂-C₁₆-alkynylphényle, C₁-C₁₆-alkoxyindole, C₃-C₆-cycloalkyle, C₁-C₁₆-alkyl-C₃-C₆-cycloalkyle, C₂-C₁₆-alkényle-C₃-C₆-cycloalkyle et C₂-C₁₆-alkynyle-C₃-C₆-cycloalkyle ; chacun de ces groupes étant éventuellement substitué par un ou plusieurs substituants choisis indépendamment les uns des autres comme étant un groupe hydroxy, amine, C₁-C₆-alkyle, C₁-C₆-hydroxyalkyle, C₁-C₆-alkoxy, C₁-C₆-thioalkyle, C₁-C₆-alkylcarbonyloxy, phényle, C₁-C₆-alkoxyphényle, ou C₂-C₆-alkénylphényle éventuellement substitué par un ou plusieurs substituants choisis indépendamment les uns des autres comme étant un groupe C₁-C₆-alkyle, C₁-C₆-hydroxyalkyle, C₁-C₆-alkoxy, C₁-C₆-thioalkyle ou C₁-C₆-alkylcarbonyloxy ;
- ou R⁵ et R⁶ forment, avec l'atome d'azote auquel ils sont reliés, un hétérocycle choisi parmi la pipéridine, la morpholine, l'hexamethyleneimine ou la pyrrolidine, éventuellement substitué par un ou plusieurs C₁-C₆-alkyle ;
à l'exception des composés suivants :
- 4-acetoxy-3,5-dimethoxy-N-morpholino-cinnamamide;
- 4-acetoxy-3,5-dimethoxy-N-(2-methylmorpholino)-cinnamamide; et
- 4-acetoxy-3,5-dimethoxy-N-(3,4,5-trimethoxyphenyl)-cinnamamide.

Les composés selon la présente invention n'ont jamais été décrits auparavant. Ces composés présentent une activité antioxydante, sont capables d'induire une augmentation significative de la néosynthèse d'élastine et/ou favorisent la néosynthèse de collagène de type I (pro-collagène I), permettant ainsi leur utilisation dans le cadre du traitement cosmétique du vieillissement cutané.

Ces composés permettent également de diminuer significativement la néo-synthèse de progérine et peuvent dont être utilisés dans la cadre du traitement thérapeutique du vieillissement cutané, notamment dans le cadre du traitement de la maladie de Hutchinson-Gilford, ou dans le traitement cosmétique du vieillissement cutané.

Les composés de l'invention permettent de diminuer le taux de glycérol dans les tissus adipeux, et sont donc utiles dans le cadre d'un traitement cosmétique amincissant. Enfin, les composés de l'invention permettent de réguler (diminuer) la synthèse de mélanine et peuvent donc être utilisés dans le cadre d'un traitement cosmétique de blanchiment de la peau.

Dans le cadre de la présente invention :
- on entend par «Cₓ-C_{y}-alkyle », une chaîne hydrocarbonée saturée, linéaire ou ramifiée, et comportant de x à y atomes de carbone ;
- on entend par « Cₓ-C_{y}-alkényle », une chaîne hydrocarbonée insaturée, linéaire ou ramifiée, contenant au moins une double liaison, et comportant de x à y atomes de carbone ;
- on entend par « Cₓ-C_{y}-alkynyle », une chaîne hydrocarbonée insaturée, linéaire ou ramifiée, contenant au moins une triple liaison, et comportant de x à y atomes de carbone ;
- on entend par « Cₓ-C_{y}-alkoxy », un groupe -O-(Cₓ-C_{y}-alkyle) ;
- on entend par « Cₓ-C_{y}-cylcoalkyle », un groupe hydrocarboné saturé qui peut être mono- ou polycyclique, et comportant de x à y atomes de carbone ;
- les termes « alkyle », « alkényle », « alkynyle », «cylcoalkyle » tels que définis ci-dessus conservent la même définition quand ils intègrent le nom d'un groupe tel que par exemple alkylthio, hydroxyalkyle, alkylphényle, alkylcarbonyloxy etc...;
- on entend par « hétérocycle » tout cycle de 5 ou 6 atomes dont un au moins est un atome d'azote, de soufre ou d'oxygène ;
- on entend par « traitement du vieillissement cutanée » tout traitement cosmétique ou thérapeutique comprenant l'application topique d'un ou plusieurs principes actifs en vue de rendre moins visibles et/ou de diminuer les signes extérieurs du vieillissement cutané tels que les rides ;
- on entend par « traitement cosmétique amincissant » tout traitement cosmétique comprenant l'application topique d'un ou plusieurs principes actifs en vue d'obtenir une réduction du volume du tissu adipeux hypodermique et/ou une réduction des vergetures ou de l'aspect « peau d'orange » du tissu cutané ;
- on entend par « traitement cosmétique de blanchiment de la peau » tout traitement cosmétique comprenant l'application topique d'un ou plusieurs principes actifs en vue d'obtenir une réduction de synthèse de mélanine, notamment le traitement cosmétique des éphélides (taches de rousseur), du chloasma (taches brunes du visage), et des pigmentations dues à la sénescence.

De préférence, la présente invention a pour objet un composé de formule générale (I) tel que défini précédemment dans lequel les caractéristiques suivantes sont choisies seules ou en combinaison :
- R¹ est choisi come étant un groupe -(C=O)-R³ ;
- R³ est choisi comme étant un groupe méthyle, éthyle, propyle, butyle, pentyle ou hexyle. De préférence encore, R³ est choisi comme étant un groupe méthyle;
- R⁴ est choisi comme étant un groupe C₁₂-C₁₆-alkyle, phényle, 4-pyranone, C₁-C₁₆-alkylphényle, C₂-C₁₆-alkénylphényle et C₃-C₆-cycloalkyle, chacun de ces groupes étant éventuellement substitué par un ou plusieurs substituants choisis indépendamment les uns des autres comme étant un groupe hydroxy, amine, C₁-C₆-alkyle, C₁-C₆-hydroxyalkyle, C₁-C₆-alkoxy, C₁-C₆-alkylthio, C₁-C₆-alkylcarbonyloxy, pipéridine, morpholine, phényle, C₁-C₆-alkoxyphényle, ou C₂-C₆-alkénylphényle éventuellement substitué par un ou plusieurs C₁-C₆-alkylcarbonyloxy. De préférence encore, R⁴ est choisi comme étant un groupe C₁₂-C₁₆-alkyle ; 4-pyranone éventuellement substitué par un ou plusieurs substituants choisis indépendamment les uns des autres comme étant un groupe C₁-C₆- hydroxyalkyle ; phényle éventuellement substitué par un ou plusieurs substituants choisis indépendamment les uns des autres comme étant C₁-C₆-alkyle ou C₁-C₆-hydroxyalkyle ; C₁-C₁₆-alkylphényle éventuellement substitué par un ou plusieurs substituants choisis indépendamment les uns des autres comme étant C₁-C₆-alkoxy, C₁-C₆-alkylthio, C₁-C₆-alkylcarbonyloxy, C₁-C₆-alkoxyphényle ou C₂-C₆-alkénylphényle lui-même éventuellement substitué par un ou plusieurs C₁-C₆-alkylcarbonyloxy ; C₂-C₁₆-alkénylphényle ; et C₃-C₆-cycloalkyle. De façon tout à fait préférée, R⁴ est choisi comme étant la 4-pyranone, le 3,4,5-trimethoxy benzyle ou un C₁₆-alkyle;
- R⁵ et R⁶ sont choisis indépendamment l'un de l'autre comme étant un atome d'hydrogène ou un groupe choisi parmi C₁-C₁₆-alkyle, C₂-C₁₆-alkényle, phényle, pipéridine, morpholine, C₁-C₁₆-alkylphényle, C₁-C₁₆-alkoxyindole et C₃-C₆-cycloalkyle, chacun de ces groupes étant éventuellement substitué par un ou plusieurs substituants choisis indépendamment les uns des autres comme étant un groupe hydroxy, amine, C₁-C₆-alkyle, C₁-C₆-hydroxyalkyle, C₁-C₆-alkoxy, C₁-C₆-thioalkyle, C₁-C₆-alkylcarbonyloxy, phényle, C₁-C₆-alkoxyphényle, ou C₂-C₆-alkénylphényle éventuellement substitué par un ou plusieurs substituants choisis indépendamment les uns des autres comme étant un groupe C₁-C₆-alkyle, C₁-C₆-hydroxyalkyle, C₁-C₆-alkoxy, C₁-C₆-thioalkyle ou C₁-C₆-alkylcarbonyloxy. De préférence encore, R⁵ et R⁶ sont choisis indépendamment l'un de l'autre comme étant un atome d'hydrogène ou un groupe choisi parmi C₁-C₁₆-alkyle ; C₂-C₁₆-alkényle ; phényle ; pipéridine ; morpholine éventuellement substituée par un ou plusieurs substituants choisis indépendamment les uns des autres comme étant un groupe hydroxy ou C₁-C₆-alkyle; C₁-C₁₆-alkylphényle éventuellement substitué par un ou plusieurs substituants choisis indépendamment les uns des autres comme étant un groupe hydroxy ou C₁-C₆-alkoxy; C₁-C₁₆-alkoxyindole ; et C₃-C₆-cycloalkyle. De façon tout à fait préférée, R⁵ est un atome d'hydrogène et R⁶ est choisi comme étant un C₁₆-alkyle ou le 4-hydroxyphenylethyl ; et/ou
- R⁵ et R⁶ forment, avec l'atome d'azote auquel ils sont reliés, un hétérocycle choisi parmi la pipéridine, la morpholine, l'hexamethyleneimine ou la pyrrolidine, éventuellement substitué par un ou plusieurs C₁-C₆-alkyle.

Les composées de formule (I) peuvent être préparés par tout procédé connu et classiquement utilisé par l'homme du métier, notamment par analogie avec les procédés décrits dans Chemische Berichte, 1952, 12, 1181.

A titre d'exemple, certains composés de formule (I) selon la présente invention pour lesquels R4 est un groupe -O-R6 peuvent être préparés :
- soit par condensation du chlorure de l'acide 4-acétoxy sinapinique (obtenu par exemple selon Monatsh, Chem 41, 271 (1920)) avec un groupe R6 tel que défini précédemment en présence d'une amine tertiaire telle que la triéthylamine, la pyridine ou la diisopropyléthylamine, à une température variant de 20°C à 155°C, et de préférence à 25°C ;
- soit par estérification de l'acide sinapinique au sein du toluène au reflux avec de l'acide sulfurique ou de l'acide méthane sulfonique comme catalyseur puis acétylation de l'ester à température ambiante dans la pyridine avec l'anhydride acétique ;
selon les schémas réactionnels suivants.

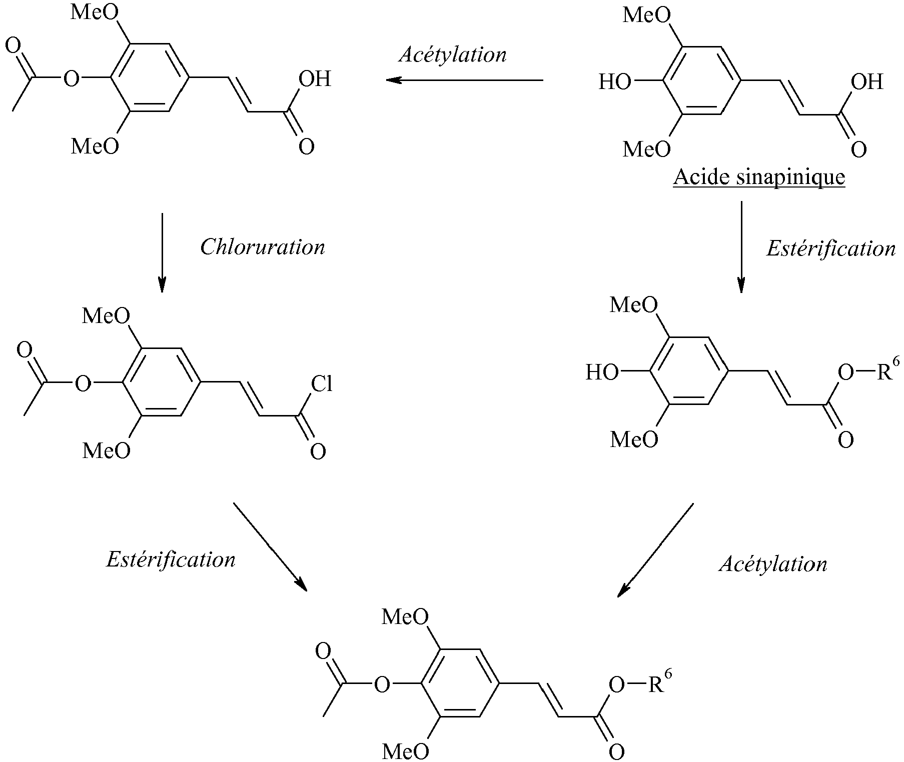

De même, certains composés de formule (I) selon la présente invention pour lesquels R⁴ est un groupe -(N)R⁷R⁸ peuvent être préparés :
- soit par condensation du chlorure de l'acide 4-acétoxy sinapinique (obtenu par exemple selon Spath, Monatsh, chem 41, 271 (1920)) avec un groupe -(N)R⁷R⁸ tel que défini précédemment, la réaction s'effectuant dans un solvant chloré tel que le chlorure de méthylène, le chloroforme ou de solvants aromatiques tels que le toluène ou le dichlorobenzene, en présence d'une amine tertiaire ou d'un large excès de l'amine réactionnelle, à une température variant de 20°C à 155°C, et de préférence à 25°C ;
- soit par réaction dans le diméthylformamide (DMF) ou le tétrahydrofurane (THF) de l'acétate de l'acide sinapinique sur une amine en présence de EDCl et d'HOBt et d'une amine tertiaire ;
- soit par une méthode dite à l'anhydride mixte, c'est à dire, par réaction de l'acétate de l'acide sinapinique dans l'acétone (ou autres solvants neutres) avec une amine tertiaire telle que la triéthylamine et un chloroformiate d'alkyle tels que le chloroformiate d'éthyle, ou le chloroformiate d'isobutyle, suivi de la réaction d'une amine primaire ou secondaire ;
selon les schémas réactionnels suivants.

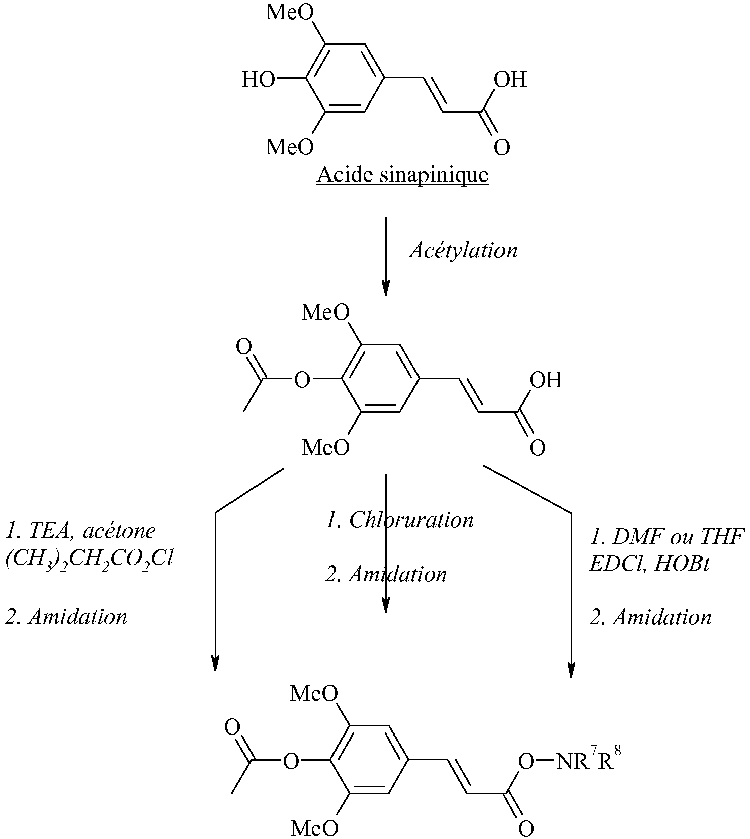

Les composés de formule (I) selon la présente invention peuvent donc être utilisés en cosmétique pour le traitement du vieillissement cutané, pour un traitement amincissant et/ou pour le blanchiment de la peau.

La présente invention a donc également pour objet l'utilisation cosmétique d'un ou plusieurs composés de formule (I) tels que définis précédemment comme agent anti-âge, comme agent amincissant et/ou comme agent de blanchiment de la peau.

La présente invention a également pour objet une composition cosmétique comprenant (à titre de principe actif) un ou plusieurs composés de formule (I) tels que définis précédemment, ainsi que son utilisation pour le traitement cosmétique du vieillissement cutané, pour un traitement cosmétique amincissant et/ou pour un traitement cosmétique de blanchiment de la peau.

Les composés de formule (I) selon la présente invention peuvent également être utilisés dans la cadre du traitement thérapeutique du vieillissement cutané, notamment dans le cadre du traitement de la maladie de Hutchinson-Gilford.

La présente invention a donc également pour objet une composition pharmaceutique comprenant (à titre de principe actif) un ou plusieurs composés de formule (I) tels que définis précédemment. De préférence, la présente invention a pour objet une composition pharmaceutique une composition pharmaceutique comprenant, à titre de principe actif, un ou plusieurs composés de formule (I) tels que définis précédemment, pour le traitement du vieillissement cutané. De façon tout à fait préférée, la présente invention a pour objet une composition pharmaceutique comprenant (à titre de principe actif) un ou plusieurs composés de formule (I) tels que définis précédemment, pour son utilisation dans le cadre du traitement de la maladie de Hutchinson-Gilford.

Les compositions cosmétiques ou pharmaceutiques selon la présente invention peuvent être formulées sous toute forme galénique appropriée à leur administration. Les compositions selon la présente invention peuvent ainsi être formulées sous forme de crème, gel, lotion, lait, émulsion huile dans eau ou eau dans huile, solution, onguent, pulvérisateur, huile corporelle, lotion après-rasage, savon, bâton protecteur des lèvres, bâton et crayon pour maquillage.

Les compositions cosmétiques ou pharmaceutiques selon la présente invention contiennent un ou plusieurs composés de formule (I) selon la présente invention dans des teneurs allant de 0,005% à 75% en poids total de la composition, préférentiellement de 0,01% à 25% en poids total de la composition, préférentiellement encore de 0,05% à 5% en poids total de la composition.

Pour la préparation de ces compositions cosmétiques ou pharmaceutiques, un ou plusieurs composés de formule (I) selon la présente invention ou un ou plusieurs de leurs sels pharmaceutiquement acceptables sont mélangés aux excipients classiquement utilisés dans le domaine cosmétique.

Les compositions cosmétiques ou pharmaceutiques selon la présente invention peuvent prendre la forme d'une crème dans laquelle un ou plusieurs composés de formule (I) selon la présente invention ou un ou plusieurs de leurs sels pharmaceutiquement acceptables sont associés aux excipients couramment utilisés en cosmétologie.
Les compositions cosmétiques ou pharmaceutiques selon la présente invention peuvent prendre la forme de gels dans les excipients appropriés tels que les esters de cellulose ou d'autres agents gélifiants, tels que le carbopol, le sepinov (polyacrylate), la gomme guar, etc.
Les compositions cosmétiques ou pharmaceutiques selon la présente invention peuvent aussi prendre la forme d'une lotion ou d'une solution dans lesquelles un ou plusieurs composés de formule (I) selon la présente invention ou un ou plusieurs de leurs sels pharmaceutiquement acceptables sont sous forme encapsulée.
Les microsphères suivant l'invention peuvent par exemple être constituées de corps gras, d'agar et d'eau. Un ou plusieurs composés de formule (I) selon la présente invention ou un ou plusieurs de leurs sels pharmaceutiquement acceptables peuvent être incorporés dans des vecteurs de type liposomes, glycosphères, cyclodextrines, dans des chylomicrons, des macro-, micro-, nano-particules ainsi que les macro-, micro- et nanocapsules et aussi être absorbés sur des polymères organiques poudreux, les talcs, bentonites et autres supports minéraux.
Ces émulsions jouissent d'une bonne stabilité et peuvent être conservées pendant le temps nécessaire pour l'utilisation à des températures comprises entre 0 et 50°C sans qu'il y ait sédimentation des constituants ou séparation des phases.
Les compositions cosmétiques ou pharmaceutiques selon la présente invention peuvent aussi contenir des additifs ou des adjuvants usuels en cosmétologies, comme par exemple des agents antimicrobiens ou des parfums mais aussi des lipides d'extraction ou de synthèse, des polymères gélifiants et viscosifiants, des tensio-actifs et des émulsifiants, des principes actifs hydro- ou liposolubles, des extraits de plantes, des extraits tissulaires, des extraits marins, des actifs de synthèse.

Les compositions cosmétiques ou pharmaceutiques selon la présente invention peuvent aussi comprendre d'autres principes actifs complémentaires choisis pour leur action. Lorsque les compositions selon la présente invention contiennent des principes actifs complémentaires, ceux-ci sont généralement présents dans la composition à une concentration suffisamment élevée pour qu'ils puissent exercer leur activité.

Les compositions cosmétiques ou pharmaceutiques selon la présente invention sont de préférence utilisées quotidiennement et appliquées une ou plusieurs fois par jour.

La présente invention est illustrée de manière non limitative par les exemples suivants.

### Exemple 1 : Composés selon l'invention

| **Composé** | **P.f. (°C)** | **GC masse** | **LCHP masse** |
|---|---|---|---|
| 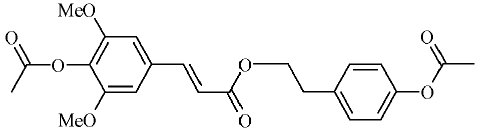 | 132 | | 428 |
| *Composé 1* | | | |
| 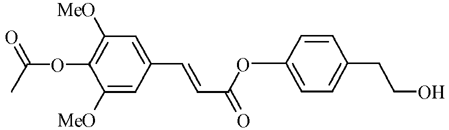 | 145 | | 386 |
| *Composé 2* | | | |
| 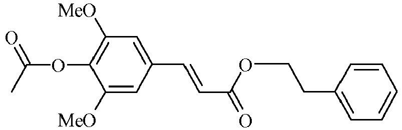 | 101 | | 370 |
| *Composé 3* | | | |
| 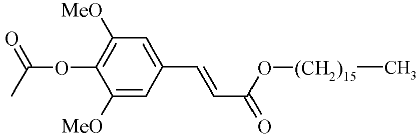 | 82 | | |
| *Composé 4* | | | |
| 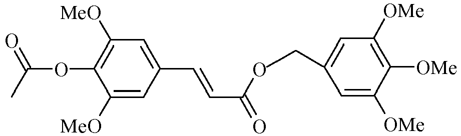 | 158 | 446 | |
| *Composé 5* | | | |
| 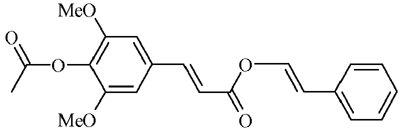 | 134 | | 368 |
| *Composé 6* | | | |
| 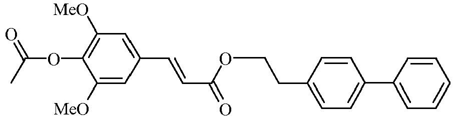 | 132 | 446 | |
| *Composé 7* | | | |
| 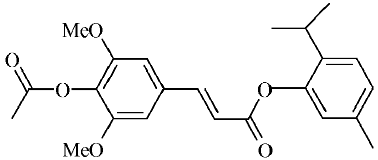 | 143 | | 398 |
| *Composé 8* | | | |
| 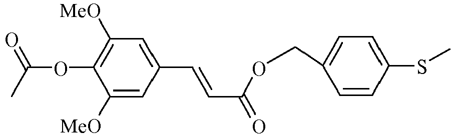 | 142 | | 402 |
| *Composé 9* | | | |
| 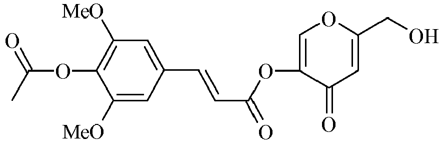 | 183 | | 390 |
| *Composé 10* | | | |
| 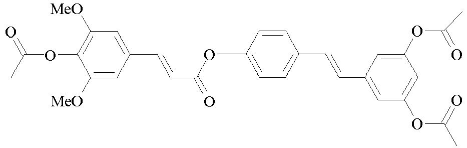 | 208 | | 560 |
| *Composé 11* | | | |
| 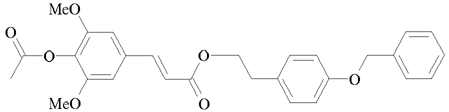 | 112 | | |
| *Composé 12* | | | |
| 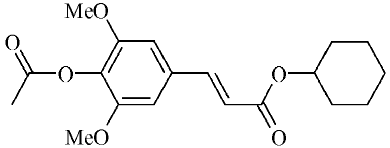 | 137 | | |
| *Composé 13* | | | |
| 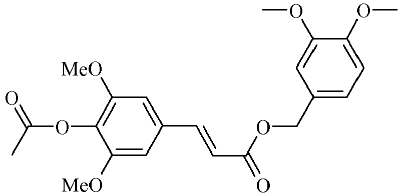 | 118 | | 416 |
| *Composé 14* | | | |
| 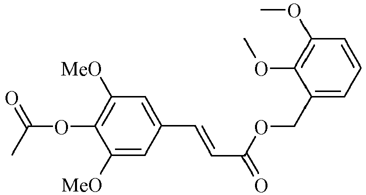 | 143 | | 416 |
| *Composé 15* | | | |
| 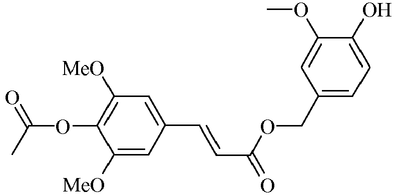 | 191 | | 402 |
| *Composé 16* | | | |
| 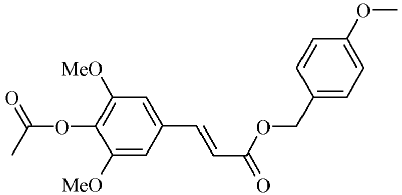 | 121 | | 386 |
| *Composé 17* | | | |
| 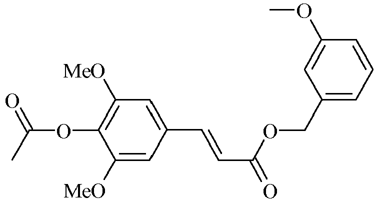 | 99 | | 386 |
| *Composé 18* | | | |
| 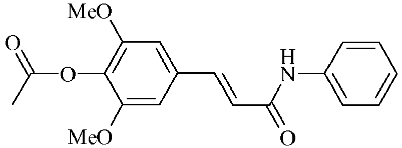 | 100 | | 341 |
| *Composé 19* | | | |
| 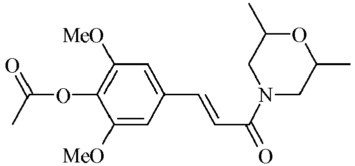 | 183 | | 363 |
| *Composé 20* | | | |
| 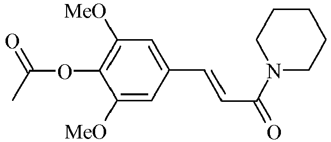 | 148 | | 333 |
| *Composé 21* | | | |
| 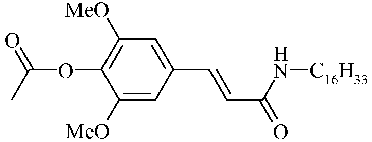 | 128 | | |
| *Composé 22* | | | |
| 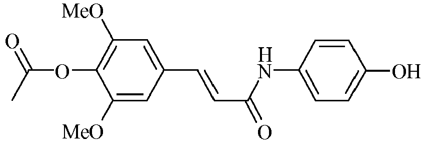 | 140 | | 357 |
| *Composé 23* | | | |
| 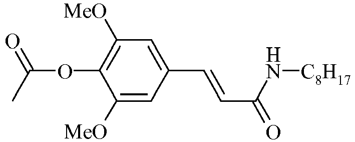 | 125 | | 377 |
| *Composé 24* | | | |
| 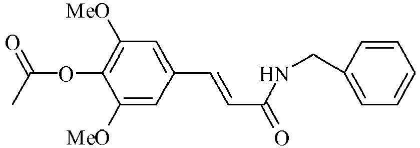 | 130 | | 355 |
| *Composé 25* | | | |
| 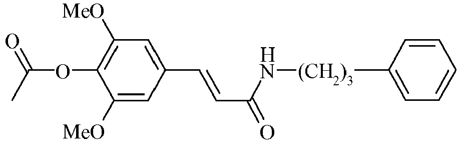 | 132 | | 383 |
| *Composé 26* | | | |
| 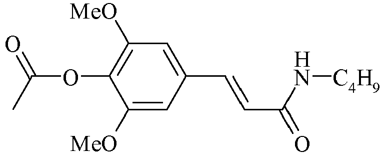 | 161 | | 321 |
| *Composé 27* | | | |
| 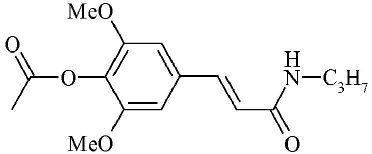 | 143 | | 307 |
| *Composé 28* | | | |
| 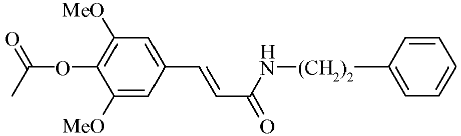 | 137 | | 369 |
| *Composé 29* | | | |
| 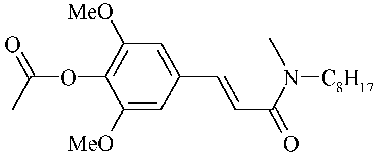 | huile | | 391 |
| *Composé 30* | | | |
| 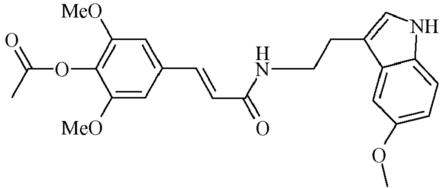 | 148 | | 438 |
| *Composé 31* | | | |
| 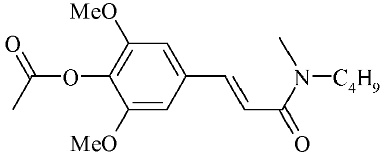 | huile | | 335 |
| *Composé 32* | | | |
| 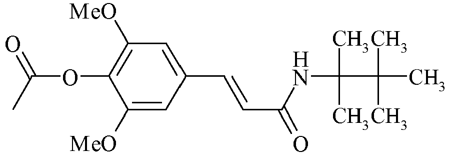 | 150 | | 377 |
| *Composé 33* | | | |
| 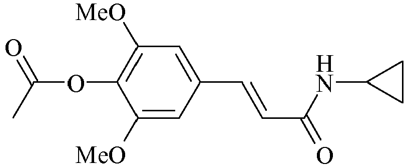 | 204 | | 305 |
| *Composé 34* | | | |
| 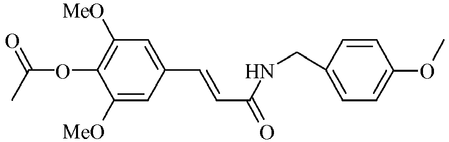 | 146 | | 385 |
| *Composé 35* | | | |
| 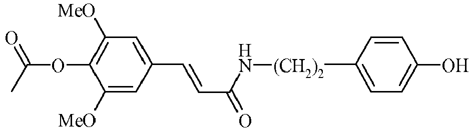 | 99 | | 385 |
| *Composé 36* | | | |
| 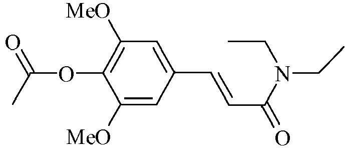 | 135 | | 321 |
| *Composé 37* | | | |
| 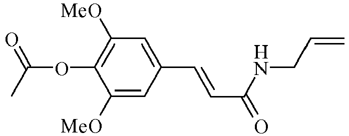 | 154 | | 305 |
| *Composé 38* | | | |
| 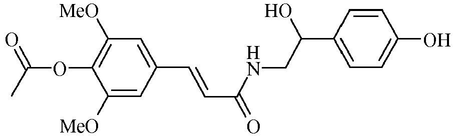 | 204 | | |
| *Composé 39* | | | |
| 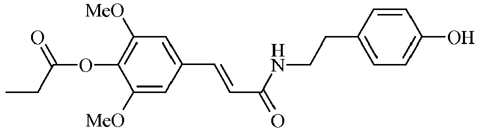 | | | 399 |
| *Composé 40* | | | |
| 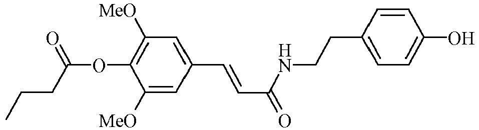 | | | 413 |
| *Composé 41* | | | |

### Exemple 2: Préparation du 4-acétoxy-3,5-diméthoxy cinnamate de 2-(4-acétoxyphenyl)-éthyle (composé 1)

### Exemple 2.1 - Préparation du chlorure d'acide de l'acétate de l'acide sinapinique

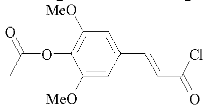

7 g de l'acétate d'acide sinapinique sont mis en suspension dans 60 ml de chloroforme. 0.5 g de dimethylformamide (DMF) sont ajoutés puis 9.4 g de chlorure de thionyle sont coulés. Le mélange est agité de 5 à 20 h à 20°C, puis chauffé à reflux pendant 3 h au moins à 8 heures au plus.

Le chloroforme et l'excès de chlorure de thionyle sont distillés sous vide à 50-60°C.

Le chlorure de l'acétate de l'acide sinapinique (p.f. : 140-144°C) est obtenu sous forme d'un produit cristallisé, jaune.

### Exemple 2.2 - Préparation du 4-acétoxy-3,5-diméthoxy cinnamate de 2-(4-acétoxyphenyl)-éthyle (composé 1)

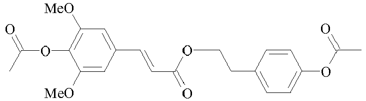

7.5 g du chlorure de l'acétate d'acide sinapinique obtenu précédemment sont mis en solution dans 60 ml de chloroforme ou de chlorure de méthylène ou de toluène. 6g de triéthylamine (ou autre amine tertiaire) puis 7.2 g (1 mol eq) de 2-(4-hydroxyphenyl) éthyle acétate (obtenu selon Tetrahedron, 56, (2000), 5169-5175) sont ajoutés.

Le mélange est agité à 20°C pendant 10h. L'évolution de la réaction est suivie par CCM (chloroforme/méthanol 9/1, révélation UV). La solution est lavée trois fois avec 100 g d'eau, puis concentrée sous vide à sec. Les cristaux obtenus sont recristallisés trois fois dans 60 ml d'éthanol absolu.

Après filtration et séchage, on obtient 2.4 g (43% de rendement) de 4-acétoxy-3,5-diméthoxy cinnamate de 2-(4-acétoxyphenyl)-éthyle (composé 1) sous forme d'une poudre blanc cassé sont isolés (p.f. : 132°C / GC/masse = 98%)

### Exemple 3 : Préparation du 4-acétoxy-3,5-diméthoxy cinnamate du 2-isopropyl-5-méthyl phényle (composé 8)

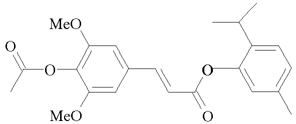

7.5 g de chlorure de l'acétate d'acide sinapinique obtenu selon l'exemple 2.1 sont mis en solution dans 60 ml de chloroforme. 8 g de pyridine et 4 g de thymol sont ajoutés.

Le mélange est agité à 20°C pendant 10h minimum. L'évolution de la réaction est suivie par CCM (chloroforme/méthanol 9/1, révélation UV). Le mélange est lavé trois fois avec 100 g d'eau. La phase organique est concentrée sous vide à sec. Le résidu obtenu est recristallisé dans l'éthanol absolu.

Après filtration et séchage, on obtient 4.8 g (46% de rendement) de 4-acétoxy-3,5-diméthoxy cinnamate du 2-isopropyl-5-méthyl phényle sous forme d'une poudre de couleur crème (p.f. 143°C / GC/masse = 98,5%)

### Exemple 4 : Préparation du 4-acétoxy-3,5-diméthoxy cinnamate de n-hexadecyle (composé 4)

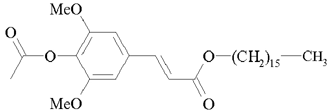

7.5 g de chlorure de l'acétate d'acide sinapinique obtenu selon l'exemple 2.1 sont mis en solution dans 60 ml de dichlorométhane. 8 g de diéthylisopropylamine et 7 g de n-hexadécanol sont ajoutés.

Le mélange est agité à 20°C pendant 20h environ. L'évolution de la réaction est suivie par CCM (chloroforme/méthanol 9/1, révélation UV). Le mélange est lavé trois fois avec 100 g d'eau. La phase organique est concentrée sous vide à sec. Le résidu obtenu est recristallisé dans l'isopropanol.

Après filtration et séchage (à 60°C), on obtient 6.0 g (44% de rendement) 4-acétoxy-3,5-diméthoxy cinnamate de n-hexadecyle sous forme d'une poudre blanc crème (p.f. : 82°C / Analyses centésimales +/- 0.3% conforme / NMR conforme)

### Exemple 5 : Préparation du 4-acétoxy-3,5-diméthoxy cinnamate de n-hexadecyle (composé 4)

### Exemple 4.1 - Préparation du 3,5-diméthoxy cinnamate de n-hexadecyle

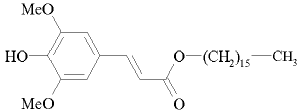

Dans un réacteur équipé d'un Dean Stark, 11.2 g d'acide sinapinique (I) sont mis en suspension dans 100 ml de toluène. 20 g de n-hexadécanol et 1 g d'acide sulfurique sont ajoutés. L'ensemble est porté à reflux pendant 5h, l'eau est soutirée.

Le milieu est refroidi vers 50°C et concentré sous vide. 120 ml d'acétate d'éthyle sont ajoutés au résidu.

La solution obtenue est lavée deux fois par 100 g d'eau, puis le solvant est concentré sous vide à 50°C. 120 ml d'heptane sont ajoutés au résidu et l'ensemble est chauffé au reflux (solubilisation) puis refroidi vers 20°C.

Les cristaux beiges sont essorés et séchés à 60°C. 20 g (rendement 89%) de 3,5-diméthoxy cinnamate de n-hexadecyle sont isolés (p.f. : 62°C / FTIR : conforme / LCHP : 98.8% / NMR conforme)

### Exemple 4.2 - Préparation du : 4-acétoxy-3,5-diméthoxy cinnamate de n-hexadecyle (composé 4)

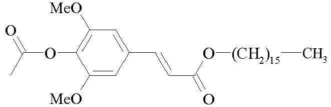

20 g de 3,5-diméthoxy cinnamate de n-hexadecyle obtenu précédemment sont dissous dans 100 g de pyridine et 28 g d'anhydride acétique sont ajoutés. L'évolution de la réaction est suivie par CCM dans le mélange chloroforme/ méthanol (9/1).

Après environ 20 heures à 20°C la solution est concentrée sous vide à 55°C. 100 ml de chlorure de méthylène sont ajoutés au résidu et la solution obtenue est lavée 2 fois par 100 g d'eau.

La phase organique est concentrée sous vide. 110 ml d'isopropanol sont ajoutés au résidu et l'ensemble est chauffé au reflux (solubilisation) puis refroidi vers 20°C.

Les cristaux formés sont essorés et séchés à 60°C. 12.8 g (rendement 57.3%) de 4-acétoxy-3,5-diméthoxy cinnamate de n-hexadecyle sous forme poudre blanche sont isolés (p.f. : 82°C / FTIR : conforme / NMR conforme).

### Exemple 6 : Préparation du 4-acétoxy-3,5-diméthoxy-N-(phenyl)-cinnamamide (composé 19)

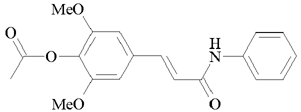

5 g de l'acétate de l'acide sinapinique sont mis en solution dans 30 ml de dimethylformamide (DMF).

4 g d'EDCI.HCl puis 2.8 g de HOBt.H₂O sont ajoutés. Le milieu est agité à 20-25°C pendant une heure.

10 g de triethylamine puis 2 g d'aniline sont ajoutés successivement au milieu. Le mélange est agité à 20°C pendant 20h. 100 g d'eau et 100 ml de chloroforme sont ajoutés, le milieu est agité 30 minutes puis laissé décanter.

La phase organique inférieure est soutirée puis lavée deux fois par 70 ml d'eau et est concentrée sous vide à sec. Le résidu huileux est repris dans 50 ml d'heptane. Le précipité formé est filtré et séché à l'étuve ventilée à 60°C.

3.2 g (rendement 50%) de 4-acétoxy-3,5-diméthoxy -N-(phenyl)-cinnamamide sous forme d'une poudre blanc cassé sont isolés (p.f. : 100°C / LCHP masse : 341 / FTIR : conforme)

### Exemple 7: Préparation de 4-acétoxy-3,5-diméthoxy-N-(cis-2,6-dimethylmorpholino)-cinnamamide (composé 20)

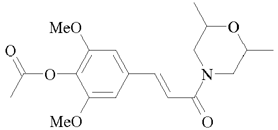

7.5 g de l'acétate d'acide sinapinique obtenu selon l'exemple 2.1 sont mis en solution dans 60 ml de chloroforme puis le mélange est refroidi à -10°C 6 g de triéthylamine puis 3 g de cis-2,6-dimethylmorpholine sont ajoutés goutte à goutte à une température inférieure à 0°C.

L'ensemble est agité à 20°C pendant 20h. Le milieu réactionnel est lavé 3 fois par 100 g d'eau. La phase organique inférieure est concentrée sous vide à sec. Le résidu est recristallisé dans l'acétate d'isopropyle. Le précipité formé est filtré et séché à l'étuve ventilée à 60°C.

5.2 g (rendement 54%) de 4-acétoxy-3,5-diméthoxy-N-(cis-2,6-dimethylmorpholino)-cinnamamide sous forme d'une poudre de couleur crème sont isolés (p.f. : 183°C / LCHP masse : 363 / FTIR : conforme)

### Exemple 8: Préparation de 4-acétoxy-3,5-diméthoxy -N-(piperidino)-cinnamamide (composé 21)

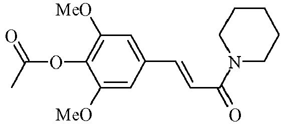

5 g de l'acétate de l'acide sinapinique sont mis en suspension dans 50 ml d'acétone. 1.9 g de triethylamine sont ajoutés. Une solution est alors obtenue.

L'ensemble est refroidi vers -10°C et 2.6 g de chloroformiate d'isobutyle sont ajoutés goutte à goutte à une température inférieure à -5°C. L'ensemble est agité une heure entre -5 et -10°C puis 1.7 g de pipéridine sont ajoutés, la température ne dépassant pas 5°C. Le milieu est agité à 20°C pendant 20h. 200 g d'eau sont alors ajoutés et le précipité formé est filtré puis séché à l'étuve ventilée à 60°C.

Le solide obtenu est recristallisé dans un minimum de méthanol. Après filtration et séchage, 3.5 g (rendement 55.5%) de 4-acétoxy-3,5-diméthoxy -N-(piperidino)-cinnamamide sous forme d'une poudre blanche sont isolés (p.f. : 148°C / LCHP masse : 333 / FTIR : conforme).

### Exemple 9: Préparation de 4-acétoxy-3,5-diméthoxy -N-(3-phenylpropyl)-cinnamamide (composé 26)

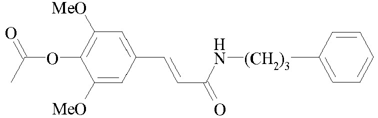

5 g de l'acétate de l'acide sinapinique sont mis en suspension dans 50 ml d'acétone. 1.9 g de triethylamine sont ajoutés.

L'ensemble est refroidi vers -10°C et 2.4 g de chloroformiate d'éthyle sont ajoutés goutte à goutte à une température inférieure à -5°C.

L'ensemble est agité une heure entre -5 et -10°C puis 2.55 g de phenyl-3-propylamine-1 sont ajoutés, la température ne dépassant pas 0°C. Le milieu est agité à 20°C pendant 12h. 2

50 g d'eau sont alors ajoutés et le précipité formé est filtré puis recristallisé dans un minimum d'éthanol à 96°. Après filtration et séchage, 4.6 g (rendement 62%) de 4-acétoxy-3,5-diméthoxy -N-(3-phenylpropyl)- cinnamamide sous forme d'une poudre blanche sont isolés (p.f. : 132°C / LCHP masse : 383 / FTIR : conforme).

### Exemple 10 : Préparation de 4-acétoxy-3,5-diméthoxy-N-(5-methoxytryptamyl)-cinnamamide (composé 31)

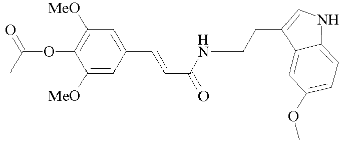

5 g de l'acétate de l'acide sinapinique sont mis en suspension dans 100 ml d'acétone. 1.7 g de triethylamine sont ajoutés.

L'ensemble est refroidi vers -10°C et 2.9 g de chloroformiate d'isobutyle sont ajoutés goutte à goutte à une température inférieure à -5°C.

L'ensemble est agité une heure entre -5 et -10°C et 3.6 g de 5-methoxytryptamine sont ensuite ajoutés, la température ne dépassant pas 0°C. Le milieu est agité à 25°C pendant 48h. 300 g d'eau sont ajoutés.

Après deux heures d'agitation le précipité formé est filtré puis recristallisé dans l'isopropanol. Après filtration et séchage, 4.2 g (rendement 51%) de sous forme d'une poudre de couleur crème sont isolés (p.f. : 148°C / LCHP masse : 438 / LCHP : 98% s/s % / FTIR : conforme)

### Exemple 11 : Préparation de 4-acétoxy-3,5-diméthoxy-N-(4'-hydroxyphenylethyl)-cinnamamide (composé 36)

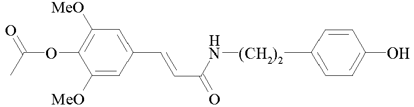

5 g de l'acétate de l'acide sinapinique sont mis en suspension dans 80 ml d'acétone. 2 g de diisopropylethylamine sont ajoutés.

L'ensemble est refroidi vers -10°C et 2.9 g de chloroformiate d'isobutyle sont ajoutés goutte à goutte à une température inférieure à -5°C. L'ensemble est agité une heure entre -5 et -10°C.

2 g de diisopropylethylamine et 3.3 g de chlorhydrate de tyramine sont ensuite ajoutés. Le milieu est agité à 20°C pendant 24h. 300 g d'eau sont ajoutés.

Après deux heures d'agitation le précipité formé est filtré puis recristallisé dans l'éthanol à 96°. Après filtration et séchage, 6.1 g (rendement 84%) de 4-acétoxy-3,5-diméthoxy-N-(4'-hydroxyphenylethyl)- cinnamamide sous forme d'une poudre légèrement colorée sont isolés (p.f. : 99°C / LCHP masse : 385 / LCHP : 98.1% s/s % FTIR : conforme).

### Exemple 12 - Activité antioxydante

La méthode ORAC (Oxygen Radical Absorbance Capacity) décrite dans Cao, G., Alessio, H. M., Cutler, R. G. Free radical Biology & Medecine 1993, 14 : 303-311 « Oxygen-Radical Absorbance Capacity assay for antioxydants » et Benderitter, M., Vincent-Genid, L., Pouget, J. P ., Voisin, P. Radiation Research 2003 ; 159 (4) : 471-483 « The cell membrane as a bioosensor of oxydative stress induced by radiation exposure : multiparameter investigation » a été utilisée pour mesurer la capacité antioxydante des composés de l'invention.

Par convention, l'activité antioxydante ORAC de toute molécule est mesurée par rapport à une référence reconnue qui est un analogue de la vitamine E, le trolox (6-hydroxy-2,5,7,8-tetramethylchroman-2-carboxylic acid), la capacité anti oxydante d'1 µM (micro mole) de trolox correspondant par définition à 1 unité ORAC.

Les valeurs ORAC sont usuellement rapportées µmol TE/g (en micro mole de trolox Equivalent par gramme de produit à tester).

Le trolox possède, compte tenu de ces définitions, un indice ORAC d'environ 3995 unités.

Les valeurs ORAC des composés 26 et 34 ont été mesurées (moyenne de 3 mesures).

Les résultats sont rapportés dans le Tableau 1 suivant.

**Tableau 1**

| **Composé** | **Activité antioxydante ORAC (µmol TE/g)** |
|---|---|
| Trolox | 3995 |
| Composé 31 | 4874 ± 216 |
| Composé 39 | 20968 ± 992 |

Ainsi, on observe que le composé 26 possède une activité antioxydante légèrement supérieure à celle du trolox, alors que le composé 34 possède une activité antioxydante 5 fois plus importante que celle du trolox.

### Exemple 13 - Effets amincissants

L'activité des composés de l'invention sur les adipocytes a été évaluée. Pour ce faire, des tests ont été réalisés sur des explants de tissus adipeux maintenus en survie selon le protocole expérimental suivant.

Les produits à tester sont introduits dans le milieu de culture. Après 9 jours de traitement, l'activité est évaluée par un dosage du glycérol libéré dans le milieu de culture.

Afin de pouvoir comparer l'activité potentielle des molécules, la caféine (Sigma réf C-0750 lot 127F0396), connue pour son activité sur les adipocytes (Studlar M. Z Ernahrungswiss. 1973 Jun;12(2):109-20, « The effect of caffeine on the metabolism of lipids and carbohydrates"), est utilisée comme actif de référence.

Les explants sont préparés à partir d'une plastie abdominale (P921-AB-40) d'un donneur féminin de 40 ans. 54 explants de tissus ont été préparés et mis en survie en milieu supplémenté en antibiotiques pénicilline/streptomicyne (Gibco réf 15140 lot 918578) à 37°C et à 5% de CO2.

Le traitement a été réalisé, sur 3 explants, par incorporation de 100 µl de produit (lui-même à 2 % de concentration dans le solvant adéquat, DMSO) dans 2 ml de milieu de culture aux jours J0, J2, J3, J5 et J7. La caféine a été diluée à la concentration voulue dans le milieu de culture pour ces mêmes jours. Les explants témoins n'ont reçu aucun traitement.

A J0, les 3 explants ont été prélevés et congelés à -80°C. A J9, les explants restants ont été prélevés et conservés dans les mêmes conditions. L'extraction des lipides du milieu de culture a été réalisée selon un mode opératoire éprouvé. Le dosage du glycérol du milieu de culture a été réalisé à l'aide du kit de dosage du glycérol (Megazyme, K-GROL) en microplaque de 96 puits et la lecture a été faite à 340 nm avec un lecteur de microplaques Tecam Infinite M200 associé au logiciel Magellan.

Les résultats obtenus sont rapportés dans le Tableau 2 suivant.

**Tableau 2**

| **Glycerol (mg/ml)** | **Moyenne** | **Écart-type** | **% par rapport à la caféine** |
|---|---|---|---|
| Milieu de culture | 0,022 | 0,005 | 4% |
| Caféine (référence) | 0,096 | 0,022 | 100% |
| Composé 5 | 0,083 | 0,037 | 82% |
| Composé 22 | 0,114 | 0,047 | 124% |

Les composés 5 et 22 possèdent donc une activité amincissante améliorée comparée à celle de la caféine, reconnue comme étant la référence en la matière. En particulier, la molécule 22 montre une action amincissante 24% supérieure à celle de la caféine.

### Exemple 14 : Effet sur la production de progérine dans des cultures monocouches de fibroblastes dermiques humains normaux issus d'un sujet mature

Afin d'étudier l'influence des composés de l'invention sur la concentration de progérine dans les cellules, la méthode de dosage décrite par C. Verdy, J.-E. Branka et N. Mekideche dans « Quantitative assessment of lactate and progerine production in normal human cutaneous cells during normal ageing : effect of an alaria esculenta extract », Int. J. Cosmetic Science, 2011, 1-5, a été utilisée.

Le système d'essai consiste en des monocouches de fibroblastes dermiques humains normaux adultes obtenues en cultivant des cellules issues d'une plastie abdominales réalisée chez une femme âgée de 54 ans.

L'insuline à 10 nM a été utilisée comme produit de référence dans cette étude.
Les cellules ont été incubées pendant 96 heures en absence (témoin) ou en présence du produit de référence ou de concentrations croissantes de l'actif à l'essai (0.01 µg/ml ; 0.1 µg/ml ; 1 µg/ml).

Le composé à tester est solubilisé dans 1 mg/ml en DMSO, puis dilué dans le milieu d'incubation des cellules, avec une concentration constante de 0.1%(v/v) en DMSO.

A la fin de la période d'incubation, d'une part, la progérine a été quantifiée dans les lysats cellulaires (obtenus par action des ultrasons) à l'aide d'un dosage ELISA, sensible et spécifique et d'autre part, les protéines contenues dans le lysats cellulaires ont été quantifiées par une méthode de spectrocolométrie (méthode de Bradford - Bradford M. (1976) Anal. Biochem., 72, 248-254).

La significativité statistique des différences observées entre les conditions « témoin » et « produit de référence » a été évaluée par un test de Student (Student *t* test). La significativité statistique des différences observées entre les conditions « Contrôle » et les « produits à l'essai » a été évalué par une analyse de la variance à un facteur (One Way ANOVA) suivie d'un test de Holm-Sidak (* : p<0,05).

Les résultats obtenus sont rapportés dans les Tableaux 3 et 4 suivants.

**Tableau 3**

| | Composé 2 | | | Composé 5 | | | Composé 10 | | |
|---|---|---|---|---|---|---|---|---|---|
| | (ng/ml) | | | (ng/ml) | | | (ng/ml) | | |
| | 10 | 100 | 500 | 10 | 100 | 500 | 50 | 500 | 5000 |
| Progérine (ng/mg de protéine) | 233,7 | 170,6 | 300,1 | 284,0 | 206,0 | 916,2 | 274,9 | 301,7 | 261,2 |
| | 353,5 | 196,3 | 328,4 | 243,7 | 364,1 | 1021,7 | 225,1 | 296,1 | 245,9 |
| | 273,6 | 402,4 | 250,5 | 391,4 | 398,6 | 657,0 | 292,3 | 271,0 | 366,9 |
| **Moyenne** | **286**,**9*** | **256**,**4*** | **293*** | **306**,**4*** | **322**,**9*** | 865,0 | **264**,**1*** | **289**,**6*** | **291**,**4*** |
| S.D. | 61,0 | 127,0 | 39,4 | 76,3 | 102,7 | 187,7 | 34,9 | 16,3 | 65,9 |
| **% DMSO** | **25,8** | **23,1** | **26,4** | **27,6** | **29,1** | **77,9** | **23,8** | **26,1** | **26,2** |
| Différence (%) | -74,2 | -76,9 | -73,6 | -72,4 | -70,9 | -22,1 | -76,2 | -73,9 | -73,8 |

| | | | | | | | | | |
|---|---|---|---|---|---|---|---|---|---|
| (*) Moyenne significativement indifférente de celle du groupe DMSO (p<0,05) | | | | | | | | | |

**Tableau 4**

| | Composé 22 | | | Composé 36 | | |
|---|---|---|---|---|---|---|
| | (ng/ml) | | | (ng/ml) | | |
| | 10 | 100 | 500 | 50 | 500 | 5000 |
| Progérine (ng/mg de protéine) | 880,7 | 711,0 | 967,8 | 1552,2 | 249,8 | 215,8 |
| | 801,5 | 548,8 | 727,4 | 690,1 | 288,8 | 200,8 |
| | 830,7 | 500,9 | 562,4 | 555,6 | 252,1 | 207,9 |
| **Moyenne** | **837,7** | **586**,**9*** | **752,5** | **932,6** | **263**,**6*** | **208**,**2*** |
| S.D. | 40,1 | 110,1 | 203,9 | 540,8 | 21,8 | 7,5 |
| **% DMSO** | **75,4** | **52,9** | **67,8** | **84,0** | **23,7** | **18,8** |
| Différence (%) | -24,6 | -47,1 | -32,2 | -16,0 | -76,3 | -81,2 |

| | | | | | | |
|---|---|---|---|---|---|---|
| (*) Moyenne significativement indifférente de celle du groupe DMSO (p<0,05) | | | | | | |

Dans les conditions expérimentales retenues les composés 2, 5, 10, 22 et 36 diminuent significativement la néosynthèse de progérine et contrebalancent ainsi le déséquilibre dans la production de progérine observée pendant le vieillissement.

### Exemple 15 : Effet sur la production de Pro-Collagène de type I dans des cultures monocouches de fibroblastes dermiques humains normaux issus d'un sujet mature

Le système d'essai consiste en des monocouches de fibroblastes dermiques humains normaux adultes obtenues en cultivant des cellules issues d'une plastie abdominales réalisée chez une femme âgée de 54 ans.
L'activateur de référence utilisé est le Transforming Growth Factor β (TGF- β) à 1 ng/ml.
Les cellules ont été incubées pendant 48 heures en absence (témoin) ou en présence du produit de référence ou de concentrations croissantes de l'actif à l'essai (10 ng/ml ; 100 ng/ml ; 500 ng/ml ou 1000 ng/ml).

Le composé à tester est solubilisé dans 1 mg/ml en DMSO, puis dilué dans le milieu d'incubation des cellules, avec une concentration constante de 0.1%(v/v) en DMSO.

A la fin de la période d'incubation, le pro-collagène de type I contenu dans les milieux d'incubation des fibroblastes a été quantifié à l'aide d'un kit E.I.A. sensible et spécifique.

A la fin de la période d'incubation, les protéines contenues dans le lysats cellulaires ont été quantifiées par une méthode de spectrocolométrie (méthode de Bradford - Bradford M. (1976) Anal. Biochem., 72, 248-254).

La significativité statistique des différences observées entre les conditions « témoin » et « produit de référence » a été évaluée par un test de Student (Student *t* test). La significativité statistique des différences observées entre les conditions « Contrôle » et les « produits à l'essai » a été évalué par une analyse de la variance à un facteur (One Way ANOVA) suivie d'un test de Holm-Sidak (* : p<0,05).

Les résultats sont donnés sous la forme de µg de pro-collagène de type I par mg de protéine (moyenne +/- la déviation standard, S.D.) et sont rapportés dans le Tableau 5 suivant.

**Tableau 5**

| | Composé 5 | | | Composé 10 | | | Composé 36 | | |
|---|---|---|---|---|---|---|---|---|---|
| | (ng/ml) | | | (ng/ml) | | | (ng/ml) | | |
| | 10 | 100 | 500 | 10 | 100 | 500 | 50 | 500 | 5000 |
| Procollagène (µg/mg de protéine) | 45,3 | 53,0 | 66,1 | 45,6 | 74,0 | 90,6 | 68,1 | 59,2 | 73,8 |
| | 37,0 | 52,3 | 62,3 | 48,7 | 75,9 | 95,4 | 57,8 | 65,1 | 64,8 |
| | 36,8 | 46,3 | 71,4 | 70,3 | 86,4 | 89,8 | 65,3 | 67,2 | 64,4 |
| **Moyenne** | **39**,**7*** | **50,5** | **66**,**6*** | **54,9** | **78**,**8*** | **91**,**9*** | **63,7*** | **63**,**9*** | **67**,**7*** |
| S.D. | 4,8 | 3,7 | 4,5 | 13,4 | 6,7 | 3,1 | 5,3 | 4,1 | 5,3 |
| **% DMSO** | **75,7** | **96,3** | **126,9** | **104,6** | **150,2** | **175,3** | **121,5** | **121,8** | **129,0** |
| Différence (%) | -24,3 | -3,7 | 26,9 | 4,6 | 50,2 | 75,3 | 21,5 | 21,8 | 29,0 |

| | | | | | | | | | |
|---|---|---|---|---|---|---|---|---|---|
| (*) Moyenne significativement indifférente de celle du groupe DMSO (p<0,05) | | | | | | | | | |

Les composés donnés en exemple induisent une augmentation significative de la néosynthèse de pro-collagène de type I, leurs conférant une activité restructurante de la matrice extracellulaire, et donc un effet anti âge de la peau.

### Exemple 16 : Effet sur la néosynthèse d'élastine dans des cultures monocouches de fibroblastes dermiques humains normaux issus d'un sujet mature

Le système d'essai consiste en des monocouches de fibroblastes dermiques humains normaux adultes obtenues en cultivant des cellules issues d'une plastie abdominales réalisée chez une femme âgée de 54 ans.

L'activateur de référence utilisé est l'acide ascorbique à 100 µg/ml.
Les cellules ont été incubées pendant 96 heures en absence (témoin) ou en présence du produit de référence ou de concentrations croissantes de l'actif à l'essai (10 ng/ml ; 100 ng/ml ; 500 ng/ml).

Le composé à tester est solubilisé dans 1 mg/ml en DMSO, puis dilué dans le milieu d'incubation des cellules, avec une concentration constante de 0.1%(v/v) en DMSO.

A la fin de la période d'incubation, l'élastine contenue dans le milieu d'incubation des explants a été quantifiée par une méthode spectrocolorimétrique.

A la fin de la période d'incubation, les protéines contenues dans le lysat cellulaire ont été quantifiées par une méthode de spectrocolométrie (méthode de Bradford - Bradford M. (1976) Anal. Biochem., 72, 248-254).

Les résultats sont donnés sous la forme de µg d'élastine par mg de protéine (moyenne +/- la déviation standard, S.D.).

A une concentration de 10 ng/ml, les composés 22 et 36 donnaient respectivement une augmentation de la néosynthèse d'élastine de +16,7% et de +63.7%.

### Exemple 17 : Effet sur la synthèse de mélanine

Ces expériences ont permis de démontrer la capacité des composés de l'invention à moduler la production de mélanine de mélanocytes humains normaux cultivés en monocouches fibroblastiques de DKK.

### Système d'essai

Des monocouches de fibroblastes dermiques humains normaux ont été obtenues en cultivant des cellules issues d'une plastie abdominale réalisée chez une femme de 54 ans. Pour la réalisation des essais, ces cellules ont été cultivées jusqu'à l'obtention de monocouches confluentes.

Des monocouches de mélanocytes humains normaux ont été obtenues en cultivant des cellules issues d'une plastie abdominale réalisée chez une femme de 41 ans. Pour la réalisation des essais, ces cellules ont été cultivées jusqu'à l'obtention de monocouches non confluentes.

### Incubation des produits

Les fibroblastes ont été incubés 48 heures à 37 °C, sous atmosphère humide et 5 % de CO2, en absence (milieu de culture seul) ou de concentrations croissantes (10; 50 ng/ml) en composé 5.

Puis les mélanocytes ont été incubés 72 heures à 37 °C, sous atmosphère humide et 5 % de CO2, en absence (milieu de culture seul), ou en présence d'acide kojique à 250 µM ou des milieux fibroblastiques conditionnés (voir ci-dessus) dilués au 1/10ème dans le milieu de culture des mélanocytes.

### Préparation de l'actif composé 5 :

L'actif a été solubilisé dans du DMSO (Diméthyl Sulfoxide).

Les dilutions ont ensuite été réalisées dans le milieu d'incubation des cellules avec une concentration constante de 0,01% (v/v) de DMSO.

### Evaluation des effets

### Dosage de la mélanine

A la fin de la période d'incubation, le contenu intracellulaire en mélanine a été quantifié dans les lysats mélanocytaires par une mesure spectrophotométrique à 405 nm.

### Dosage des protéines

A la fin de la période d'incubation, les protéines contenues dans les lysats cellulaires ont été quantifiées par une méthode spectrocolorimétrique (méthode de Bradford).

### Résultats & Statistiques

Les résultats sont donnés sous la forme de µg de mélanine intracellulaire par mg de protéines totales du tapis cellulaire (moyennes +/- la déviation standard, S.D.).

La significativité statistique des différences observées entre la condition «Témoin» et « produit à l'essai », a été évaluée, par une analyse de la variance à un facteur (One Way ANOVA) suivie d'un test de Holm-Sidak (* : p<0.05).

Les résultats obtenus sont rapportés dans les tableaux 6 et 7 suivants.

**Tableau 6**

| | **Contrôle** | **Acide Kojique 250 µM** | **DMSO 0,1 %** |
|---|---|---|---|
| Mélanine / Protéines (µg/mg) | 16,7 | 14,3 | 15,8 |
| | 15,2 | 13,4 | 17,0 |
| | 16,5 | 14,1 | 18,1 |
| **Moyenne** | **16,1** | **14,0** | **17,0** |
| S.D. | 0,8 | 0,5 | 1,1 |
| **% témoin** | **100,0** | **86**,**6*** | **105,1** |

| | | | |
|---|---|---|---|
| (*) Moyenne significativement indifférente de celle du DMSO 0.1% (p<0,05) | | | |

**Tableau 7**

| | **Composé 5** | | | **DMSO 0,01%** |
|---|---|---|---|---|
| | **10 ng/ml** | **50 ng/ml** | **100 ng/ml** | |
| Mélanine / Protéines (µg/mg) | 14,2 | 14,3 | 15,8 | 16,1 |
| | 11,7 | 14,7 | 16,7 | 17,3 |
| | 13,7 | 14,7 | 16,0 | 18,4 |
| **Moyenne** | **14**,**3*** | **14**,**4*** | 16,2 | 17,3 |
| S.D. | 1,3 | 0,2 | 0,5 | 1,1 |
| **% DMSO** | **83,0** | **83,6** | **93,7** | 100,0 |
| Différence (%) | -17,0 | -16,4 | -6,3 | |

| | | | | |
|---|---|---|---|---|
| (*) Moyenne significativement indifférente de celle du DMSO 0.1% (p<0,05) | | | | |

Ces résultats démontrent que le composé 5 diminue la synthèse de mélanine dans les mélanocytes de façon significative, ce qui lui confère une activité blanchissante.

## Revendications

1. Composé de formule générale (I) 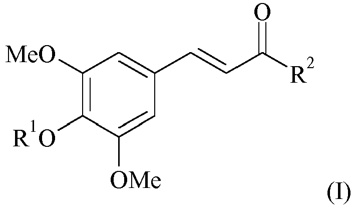 dans laquelle :
- R¹ est choisi comme étant un groupe C₂-C₆-alkyle ou un groupe -(C=O)-R³;
- R² est choisi comme étant un groupe -O-R⁴ ou -(N)R⁵R⁶ ;
- R³ est choisi comme étant un groupe C₁-C₆-alkyle ;
- R⁴ est choisi comme étant un groupe C₁₂-C₁₆-alkyle, C₁₂-C₁₆-alkényle, C₁₂-C₁₆-alkynyle, phényle, 4-pyranone, C₁-C₁₆-alkylphényle, C₂-C₁₆-alkénylphényle, C₂-C₁₆-alkynylphényle, C₃-C₆-cycloalkyle, C₁-C₁₆-alkyl-C₃-C₆-cycloalkyle, C₂-C₁₆-alkényle-C₃-C₆-cycloalkyle et C₂-C₁₆-alkynyle-C₃-C₆-cycloalkyle ; chacun de ces groupes étant éventuellement substitué par un ou plusieurs substituants choisis indépendamment les uns des autres comme étant un groupe hydroxy, amine, C₁-C₆-alkyle, C₁-C₆-hydroxyalkyle, C₁-C₆-alkoxy, C₁-C₆-alkylthio, C₁-C₆-alkylcarbonyloxy, phényle, C₁-C₆-alkoxyphényle, ou C₂-C₆-alkénylphényle éventuellement substitué par un ou plusieurs substituants choisis indépendamment les uns des autres comme étant un groupe C₁-C₆-alkyle, C₁-C₆-hydroxyalkyle, C₁-C₆-alkoxy, C₁-C₆-thioalkyle ou C₁-C₆-alkylcarbonyloxy ;
- R⁵ et R⁶ sont choisis indépendamment l'un de l'autre comme étant un atome d'hydrogène ou un groupe choisi parmi C₁-C₁₆-alkyle, C₂-C₁₆-alkényle, C₂-C₁₆-alkynyle, phényle, C₁-C₁₆-alkylphényle, C₂-C₁₆-alkénylphényle, C₂-C₁₆-alkynylphényle, C₁-C₁₆-alkoxyindole, C₃-C₆-cycloalkyle, C₁-C₁₆-alkyl-C₃-C₆-cycloalkyle, C₂-C₁₆-alkényle-C₃-C₆-cycloalkyle et C₂-C₁₆-alkynyle-C₃-C₆-cycloalkyle ; chacun de ces groupes étant éventuellement substitué par un ou plusieurs substituants choisis indépendamment les uns des autres comme étant un groupe hydroxy, amine, C₁-C₆-alkyle, C₁-C₆-hydroxyalkyle, C₁-C₆-alkoxy, C₁-C₆-thioalkyle, C₁-C₆-alkylcarbonyloxy, phényle, C₁-C₆-alkoxyphényle, ou C₂-C₆-alkénylphényle éventuellement substitué par un ou plusieurs substituants choisis indépendamment les uns des autres comme étant un groupe C₁-C₆-alkyle, C₁-C₆-hydroxyalkyle, C₁-C₆-alkoxy, C₁-C₆-thioalkyle ou C₁-C₆-alkylcarbonyloxy ;
- ou R⁵ et R⁶ forment, avec l'atome d'azote auquel ils sont reliés, un hétérocyclechoisi parmi la pipéridine, la morpholine, l'hexamethyleneimine ou la pyrrolidine, éventuellement substitué par un ou plusieurs C₁-C₆-alkyle ;
à l'exception des composés suivants :
- 4-acetoxy-3,5-dimethoxy-N-morpholino-cinnamamide;
- 4-acetoxy-3,5-dimethoxy-N-(2-methylmorpholino)-cinnamamide; et
- 4-acetoxy-3,5-dimethoxy-N-(3,4,5-trimethoxyphenyl)-cinnamamide.

2. Composé selon la revendication 1, **caractérisé en ce que** R¹ est choisi come étant un groupe -(C=O)-R⁵.

3. Composé selon la revendication 1 ou 2, **caractérisé en ce que** R³ est choisi comme étant un groupe méthyle, éthyle, propyle, butyle, pentyle ou hexyle.

4. Composé selon l'une quelconque des revendications 1 à 3, **caractérisé en ce que** R⁴ est choisi comme étant un groupe C₁₂-C₁₆-alkyle, phényle, 4-pyranone, C₁-C₁₆-alkylphényle, C₂-C₁₆-alkénylphényle et C₃-C₆-cycloalkyle, chacun de ces groupes étant éventuellement substitué par un ou plusieurs substituants choisis indépendamment les uns des autres comme étant un groupe hydroxy, amine, C₁-C₆-alkyle, C₁-C₆-hydroxyalkyle, C₁-C₆-alkoxy, C₁-C₆-alkylthio, C₁-C₆-alkylcarbonyloxy, pipéridine, morpholine, phényle, C₁-C₆-alkoxyphényle, ou C₂-C₆-alkénylphényle éventuellement substitué par un ou plusieurs C₁-C₆-alkylcarbonyloxy..

5. Composé selon la revendication 4, **caractérisé en ce que** R⁴ est choisi comme étant un groupe C₁₂-C₁₆-alkyle ; 4-pyranone éventuellement substitué par un ou plusieurs substituants choisis indépendamment les uns des autres comme étant un groupe C₁-C₆- hydroxyalkyle ; phényle éventuellement substitué par un ou plusieurs substituants choisis indépendamment les uns des autres comme étant C₁-C₆-alkyle ou C₁-C₆-hydroxyalkyle ; C₁-C₁₆-alkylphényle éventuellement substitué par un ou plusieurs substituants choisis indépendamment les uns des autres comme étant C₁-C₆-alkoxy, C₁-C₆-alkylthio, C₁-C₆-alkylcarbonyloxy, C₁-C₆-alkoxyphényle ou C₂-C₆-alkénylphényle lui-même éventuellement substitué par un ou plusieurs C₁-C₆-alkylcarbonyloxy ; C₂-C₁₆-alkénylphényle ; et C₃-C₆-cycloalkyle.

6. Composé selon la revendication 5, **caractérisé en ce que** R⁴ est choisi comme étant la 4-pyranone, le 3,4,5-trimethoxy benzyle ou un C₁₆-alkyle.

7. Composé selon l'une quelconque des revendications 1 à 6, **caractérisé en ce que** R⁵ et R⁶ sont choisis indépendamment l'un de l'autre comme étant un atome d'hydrogène ou un groupe choisi parmi C₁-C₁₆-alkyle, C₂-C₁₆-alkényle, phényle, pipéridine, morpholine, C₁-C₁₆-alkylphényle, C₁-C₁₆-alkoxyindole et C₃-C₆-cycloalkyle, chacun de ces groupes étant éventuellement substitué par un ou plusieurs substituants choisis indépendamment les uns des autres comme étant un groupe hydroxy, amine, C₁-C₆-alkyle, C₁-C₆-hydroxyalkyle, C₁-C₆-alkoxy, C₁-C₆-thioalkyle, C₁-C₆-alkylcarbonyloxy, phényle, C₁-C₆-alkoxyphényle, ou C₂-C₆-alkénylphényle éventuellement substitué par un ou plusieurs substituants choisis indépendamment les uns des autres comme étant un groupe C₁-C₆-alkyle, C₁-C₆-hydroxyalkyle, C₁-C₆-alkoxy, C₁-C₆-thioalkyle ou C₁-C₆-alkylcarbonyloxy.

8. Composé selon la revendication 7, **caractérisé en ce que** R⁵ et R⁶ sont choisis indépendamment l'un de l'autre comme étant un atome d'hydrogène ou un groupe choisi parmi C₁-C₁₆-alkyle ; C₂-C₁₆-alkényle ; phényle ; pipéridine ; morpholine éventuellement substituée par un ou plusieurs substituants choisis indépendamment les uns des autres comme étant un groupe hydroxy ou C₁-C₆-alkyle; C₁-C₁₆-alkylphényle éventuellement substitué par un ou plusieurs substituants choisis indépendamment les uns des autres comme étant un groupe hydroxy ou C₁-C₆-alkoxy; C₁-C₁₆-alkoxyindole ; et C₃-C₆-cycloalkyle.

9. Composé selon la revendication 8, **caractérisé en ce que** R⁵ est un atome d'hydrogène et R⁶ est choisi comme étant un groupe C₁₆-alkyle ou le 4-hydroxyphenylethyl

10. Composition cosmétique comprenant un composé selon l'une quelconque des revendications 1 à 9.

11. Composition selon la revendication 10 pour le traitement cosmétique du vieillissement cutané, pour un traitement cosmétique amincissant et/ou pour un traitement cosmétique de blanchiment de la peau.

12. Composition pharmaceutique comprenant un composé selon l'une quelconque des revendications 1 à 9.

13. Composition selon la revendication 12 pour son utilisation dans le cadre du traitement de la maladie de Hutchinson-Gilford.

## Patentansprüche

1. Verbindung mit der allgemeinen Formel (I) 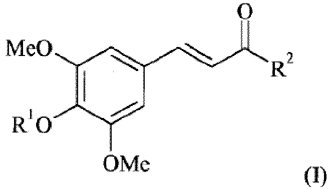 wobei:
- R¹ ausgewählt ist als eine Gruppe C₂-C₆-Alkyl oder eine Gruppe - (C=O)-R³;
- R² ausgewählt ist als eine Gruppe -O-R⁴ oder -(N)R⁵R⁶;
- R³ ausgewählt ist als eine Gruppe C₁-C₆-Alkyl;
- R⁴ ausgewählt ist als eine Gruppe C₁₂-C₁₆-Alkyl, C₁₂-C₁₆-Alkenyl, C₁₂-C₁₆-Alkynyl, Phenyl, 4-Pyranon, C₁ -C₁₆-Alkylphenyl, C₂-C₁₆-Alkenylphenyl, C₂-C₁₆-Alkynylphenyl, C₃-C₆-Cycloalkyl, C₁-C₁₆-Alkyl-C₃-C₆-cycloalkyl, C₂-C₁₆-Alkenyl-C₃-C₆-cycloalkyl und C₂-C₁₆-Alkynyl-C₃-C₆-cycloalkyl; wobei jede dieser Gruppen eventuell substituiert ist durch einen oder mehrere Substituenten, ausgewählt unabhängig voneinander als eine Gruppe Hydroxy, Amino, C₁-C₆-Alkyl, C₁-C₆-Hydroxyalkyl, C₁-C₆-Alkoxy, C₁-C₆-Alkylthio, C₁-C₆-Alkylcarbonyloxy, Phenyl, C₁-C₆-Alkoxyphenyl oder C₂-C₆-Alkenylphenyl, eventuell substituiert durch einen oder mehrere Substituenten, ausgewählt unabhängig voneinander als eine Gruppe C₁-C₆-Alkyl, C₁-C₆-Hydroxyalkyl, C₁-C₆-Alkoxy, C₁-C₆-Thioalkyl oder C₁-C₆-Alkylcarbonyloxy;
- R⁵ und R⁶ unabhängig voneinander ausgewählt sind als ein Wasserstoffatom oder eine Gruppe, ausgewählt aus C₁-C₁₆-Alkyl, C₂-C₁₆-Alkenyl, C₂-C₁₆-Alkynyl, Phenyl, C₁-C₁₆-Alkylphenyl, C₂-C₁₆-Alkenylphenyl, C₂-C₁₆-Alkynylphenyl, C₁-C₁₆-Alkoxyindol, C₃-C₆-Cycloalkyl, C₁-C₁₆-Alkyl-C₃-C₆-cycloalkyl, C₂-C1₆-Alkenyl-C₃-C₆-Cycloalkyl und C₂-C₁₆-Alkynyl-C₃-C₆-cycloalkyl; wobei jede dieser Gruppen eventuell substituiert ist durch einen oder mehrere Substituenten, ausgewählt unabhängig voneinander als eine Gruppe Hydroxy, Amino,C₁-C₆-Alkyl, C₁-C₆-Hydroxyalkyl, C₁-C₆-Alkoxy, C₁-C₆-Thioalkyl, C₁-C₆-Alkylcarbonyloxy, Phenyl, C₁-C₆-Alkoxyphenyl oder C₂-C₆-Alkenylphenyl, eventuell substituiert durch einen oder mehrere Substituenten, ausgewählt unabhängig voneinander als eine Gruppe C₁-C₆-Alkyl, C₁-C₆-Hydroxyalkyl, C₁-C₆-Alkoxy, C₁-C₆-Thioalkyl oder C₁-C₆-Alkylcarbonyloxy;
- oder R⁵ und R⁶ mit dem Stickstoffatom, mit dem sie verbunden sind, einen Heterocyclus bilden, ausgewählt aus Piperidin, Morpholin, Hexamethylenimin oder Pyrrolidin, eventuell substituiert durch ein oder mehrere C₁-C₆-Alkyl;
mit Ausnahme der folgenden Verbindungen:
- 4-Acetoxy-3,5-dimethoxy-N-morpholino-cinnamamid;
- 4-Acetoxy-3,5-dimethoxy-N-(2-methylmorpholino)-cinnamamid; und
- 4-Acetoxy-3,5-dimethoxy-N-(3,4,5-trimethoxyphenyl)-cinnamamid.

2. Verbindung nach Anspruch 1, **dadurch gekennzeichnet, dass** R¹ ausgewählt ist als eine Gruppe -(C=O)-R⁵.

3. Verbindung nach Anspruch 1 oder 2, **dadurch gekennzeichnet, dass** R³ ausgewählt ist als eine Gruppe Methyl, Ethyl, Propyl, Butyl, Pentyl oder Hexyl.

4. Verbindung nach einem der Ansprüche 1 bis 3, **dadurch gekennzeichnet, dass** R⁴ ausgewählt ist als eine Gruppe C₁₂-C₁₆-Alkyl, Phenyl, 4-Pyranon, C₁-C₁₆-Alkylphenyl, C₂-C₁₆-Alkenylphenyl und C₃-C₆-Cycloalkyl, wobei jede dieser Gruppen eventuell substituiert ist durch einen oder mehrere Substituenten, ausgewählt unabhängig voneinander als eine Gruppe Hydroxy, Amino, C₁-C₆-Alkyl, C₁-C₆-Hydroxyalkyl, C₁-C₆-Alkoxy, C₁-C₆-Alkylthio, C₁-C₆-Alkylcarbonyloxy, Piperidin, Morpholin, Phenyl, C₁-C₆-Alkoxyphenyl oder C₂-C₆-Alkenylphenyl, eventuell substituiert durch ein oder mehrere C₁-C₆-Alkylcarbonyloxy;

5. Verbindung nach Anspruch 4, **dadurch gekennzeichnet, dass** R⁴ ausgewählt ist als eine Gruppe C₁₂-C₁₆-Alkyl, 4-Pyranon, eventuell substituiert durch einen oder mehrere Substituenten, ausgewählt unabhängig voneinander als eine Gruppe C₁-C₆-Hydroxyalkyl; Phenyl, eventuell substituiert durch einen oder mehrere Substituenten, ausgewählt unabhängig voneinander als C₁-C₆-Alkyl oder C₁-C₆-Hydroxyalkyl; C₁-C₁₆-Alkylphenyl, eventuell substituiert durch einen oder mehrere Substituenten, ausgewählt unabhängig voneinander als C₁-C₆-Alkoxy, C₁-C₆-Alkylthio, C₁-C₆-Alkylcarbonyloxy, C₁-C₆-Alkoxyphenyl oder C₂-C₆-Alkenylphenyl, selbst eventuell substituiert durch ein oder mehrere C₁-C₆-Alkylcarbonyloxy; C₂-C₁₆-Alkenylphenyl; und C₃-C₆-Cycloalkyl.

6. Verbindung nach Anspruch 5, **dadurch gekennzeichnet, dass** R⁴ ausgewählt ist als 4-Pyranon, 3,4,5-Trimethoxybenzyl oder ein C₁₆-Alkyl.

7. Verbindung nach einem der Ansprüche 1 bis 6, **dadurch gekennzeichnet, dass** R⁵ und R⁶ unabhängig ausgewählt sind als ein Wasserstoffatom, oder eine Gruppe, ausgewählt aus C₁-C₁₆-Alkyl, C₂-C₁₆-Alkenyl, Phenyl, Piperidin, Morpholin, C₁-C₁₆-Alkylphenyl, C₁-C₁₆-Alkoxyindol und C₃-C₆-Cycloalkyl, wobei jede dieser Gruppen eventuell substituiert ist durch einen oder mehrere Substituenten, ausgewählt unabhängig voneinander als eine Gruppe Hydroxy, Amino, C₁-C₆-Alkyl, C₁-C₆-Hydroxyalkyl, C₁-C₆-Alkoxy, C₁-C₆-Thioalkyl, C₁-C₆-Alkylcarbonyloxy, Phenyl, C₁-C₆-Alkoxyphenyl oder C₂-C₆-Alkenylphenyl, eventuell substituiert durch einen oder mehrere Substituenten, ausgewählt unabhängig voneinander als eine Gruppe C₁-C₆-Alkyl, C₁-C₆-Hydroxyalkyl, C₁-C₆-Alkoxy, C₁-C₆-Thioalkyl oder C₁-C₆-Alkylcarbonyloxy.

8. Verbindung nach Anspruch 7, **dadurch gekennzeichnet, dass** R⁵ und R⁶ unabhängig voneinander ausgewählt sind als ein Wasserstoffatom, oder eine Gruppe, ausgewählt aus C₁-C₁₆-Alkyl; C₂-C₁₆-Alkenyl; Phenyl; Piperidin; Morpholin, eventuell substituiert durch einen oder mehrere Substituenten, ausgewählt unabhängig voneinander als eine Gruppe Hydroxy, oder C₁-C₆-Alkyl; C₁-C₁₆-Alkylphenyl, eventuell substituiert durch einen oder mehrere Substituenten, ausgewählt unabhängig voneinander als eine Gruppe Hydroxy, oder C₁-C₆-Alkoxy; C₁-C₁₆-Alkoxyindol; und C₃-C₆-Cycloalkyl.

9. Verbindung nach Anspruch 8, **dadurch gekennzeichnet, dass** R⁵ ein Wasserstoffatom ist und R⁶ ausgewählt ist als eine Gruppe C₁₆-Alkyl oder 4-Hydroxyphenylethyl.

10. Kosmetische Zusammensetzung, umfassend eine Verbindung nach einem der Ansprüche 1 bis 9.

11. Zusammensetzung nach Anspruch 10 zur kosmetischen Behandlung der Alterung der Haut, für eine kosmetische Verschlankungsbehandlung und/oder eine kosmetische Behandlung zur Bleichung der Haut.

12. Kosmetische Zusammensetzung, umfassend eine Verbindung nach einem der Ansprüche 1 bis 9.

13. Zusammensetzung nach Anspruch 12, für ihre Verwendung im Rahmen der Behandlung der Hutchinson-Gilford-Erkrankung.

## Claims

1. A compound of general formula (I) 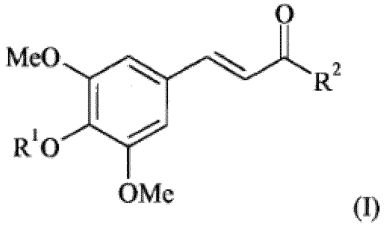 wherein:
- R¹ is selected as a C₂-C₆-alkyl group or a -(C=O)-R³ group;
- R² is selected as an -O-R⁴ or a -(N)R⁵R⁶ group;
- R³ is selected as a C₁-C₆-alkyl group;
- R⁴ is selected as a C₁₂-C₁₆-alkyl, C₁₂-C₁₆-alkenyl, C₁₂-C₁₆-alkynyl, phenyl, 4-pyranone, C₁-C₁₆-alkylphenyl, C₂-C₁₆-alkenylphenyl, C₃-C₆-cycloalkyl, C₁-C₁₆-alkyl-C₃-C₆-cycloalkyl, C₂-C₁₆-alkenyl-C₃-C₆-cycloalkyl and a C₂-C₁₆-alkynyl-C₃-C₆-cycloalkyl group; each of these groups being optionally substituted by one or more substituent(s) selected independently of each other as an hydroxy, amine, C₁-C₆-alkyl, C₁-C₆-hydroxyalkyl, C₁-C₆-alkoxy, C₁-C₆-alkylthio, C₁-C₆-alkylcarbonyloxy, phenyl, C₁-C₆-alkoxyphenyl, or C₂-C₆-alkenylphenyl group optionally substituted by one or more substituent(s) selected independently of each other as a C₁-C₆-alkyl, C₁-C₆-hydroxyalkyl, C₁-C₆-alkoxy, C₁-C₆-thioalkyl or a C₁-C₆-alkylcarbonyloxy group;
- R⁵ and R⁶ are selected independently of each other as a hydrogen atom or a group selected from a C₁-C₁₆-alkyl, C₂-C₁₆-alkenyl, C₂-C₁₆-alkynyl, phenyl, C₁-C₁₆-alkylphenyl, C₂-C₁₆-alkenylphenyl, C₂-C₁₆-alkynylphenyl, C₁-C₁₆-alkoxyindole, C₃-C₆-cycloalkyl, C₁-C₁₆-alkyl-C₃-C₆-cycloalkyl, C₂-C₁₆-alkenyl-C₃-C₆-cycloalkyl and a C₂-C₁₆-alkynyl-C₃-C₆-cycloalkyl group; each of these groups being optionally substituted by one or more substituent(s) selected independently of each other as a hydroxy, amine, C₁-C₆-alkyl, C₁-C₆-hydroxyalkyl, C₁-C₆-alkoxy, C₁-C₆-thioalkyl, C₁-C₆-alkylcarbonyloxy, phenyl, C₁-C₆-alkoxyphenyl, or a C₂-C₆-alkenylphenyl optionally substituted by one or more substituent(s) selected independently of each other from a C₁-C₆-alkyl, C₁-C₆-hydroxyalkyl, C₁-C₆-alkoxy, C₁-C₆-thioalkyl or a C₁-C₆-alkylcarbonyloxy group;
- or R⁵ and R⁶ form, with the nitrogen atom to which they are linked, a heterocycle selected from piperidine, morpholine, hexamethyleneimine or the pyrrolidine, optionally substituted by one or more C₁-C₆-alkyl group(s);
with the exception of the following compounds:
- 4-acetoxy-3,5-dimethoxy-N-morpholino-cinnamamide;
- 4-acetoxy-3,5-dimethoxy-N-(2-methylmorpholino)-cinnamamide;
- 4-acetoxy-3,5-dimethoxy-N-(3,4,5-trimethoxyphenyl)-cinnamamide.

2. The compound according to claim 1, **characterized in that** R¹ is chosen as being a -(C=O)-R⁵ group.

3. The compound according to claim 1 or 2, **characterized in that** R³ is chosen as being a methyl, ethyl, propyl, butyl, pentyl or hexyl group.

4. The compound according to any one of claims 1 to 3, **characterized in that** R⁴ is chosen as being a C₁₂-C₁₆-alkyl, phenyl, 4-pyranone, C₁-C₁₆-alkylphenyl, C₂-C₁₆-alkenylphenyl and a C₃-C₆-cycloalkyl group, each of these groups being optionally substituted by one or more substituent(s) selected independently of each other from a hydroxy, amine, C₁-C₆-alkyl, C₁-C₆-hydroxyalkyl, C₁-C₆-alkoxy, C₁-C₆-alkylthio, C₁-C₆-alkylcarbonyloxy, piperidine, morpholine, phenyl, C₁-C₆-alkoxyphenyl, or a C₂-C₆-alkenylphenyl group optionally substituted by one or more C₁-C₆-alkylcarbonyloxy group(s).

5. The compound according to claim 4, **characterized in that** R⁴ is chosen as being a C₁₂-C₁₆-alkyl; 4-pyranone group optionally substituted by one or more substituents(s) selected independently of each other as a C₁-C₆-hydroxyalkyl; phenyl group optionally substituted by one or more substituent(s) selected independently of each other as a C₁-C₆-alkyl or a C₁-C₆-hydroxyalkyl; C₁-C₁₆-alkylphenyl group optionally substituted by one or more substituent(s) selected independently of each other as a C₁-C₆-alkoxy, C₁-C₆-alkylthio, C₁-C₆-alkylcarbonyloxy; C₁-C₆-alkoxyphenyl or a C₂-C₆-alkenylphenyl group itself optionally substituted by one or more C₁-C₆-alkylcarbonyloxy; C₂-C₁₆-alkenylphenyl; and C₃-C₆-cycloalkyl group(s).

6. The compound according to claim 5, **characterized in that** R⁴ is chosen as being the 4-pyranone group, the 3,4,5-trimethoxybenzyl or a C₁₆-alkyl group.

7. The compound according to any one of claims 1 to 6, **characterized in that** R⁵ and R⁶ are chosen independently of each other as being a hydrogen atom or a group selected from a C₁-C₁₆-alkyl, C₂-C₁₆-alkenyl, phenyl, piperidine, morpholine, C₁-C₁₆-alkylphenyl, C₁-C₁₆-alkoxyindole and C₃-C₆-cycloalkyl group, each of these groups being optionally substituted by one or more substituent(s) selected independently of each other as a hydroxy, amine, C₁-C₆-alkyl, C₁-C₆-hydroxyalkyl, C₁-C₆-alkoxy, C₁-C₆-thioalkyl, C₁-C₆-alkylcarbonyloxy, phenyl, C₁-C₆-alkoxyphenyl, or a C₂-C₆-alkenylphenyl group optionally substituted by one or more substituent(s) selected independently of each other as a C₁-C₆-alkyl, C₁-C₆-hydroxyalkyl, C₁-C₆-alkoxy, C₁-C₆-thioalkyl, or a C₁-C₆-alkylcarbonyloxy group.

8. The compound according to claim 7, **characterized in that** R⁵ and R⁶ are chosen independently of each other as being a hydrogen atom or a group selected from a C₁-C₁₆-alkyl; C₂-C₁₆-alkenyl; phenyl; piperidine; morpholine group optionally substituted by one or more substituent(s) selected independently of each other as a hydroxy or a C₁-C₆-alkyl; C₁-C₁₆-alkylphenyl group optionally substituted by one or more substituent(s) selected independently of each other as a hydroxy or a C₁-C₆-alkoxy; C₁-C₁₆-alkoxyindole; and a C₃-C₆-cycloalkyl group.

9. The compound according to claim 8, **characterized in that** R⁵ is a hydrogen atom and R⁶ is chosen as being a C₁₆-alkyl group or 4-hydroxyphenylethyl group.

10. A cosmetic composition comprising a compound according to any one of claims 1 to 9.

11. The composition according to claim 10 for the cosmetic treatment of the cutaneous ageing, for a slimming cosmetic treatment and/or a skin cosmetic whitening treatment.

12. The pharmaceutical composition comprising a compound according to any one of claims 1 to 9.

13. The composition according to claim 12, for its use in the treatment of the Hutchinson-Gilford disease.
